(19) Europäisches Patentamt
European Patent Office
Office européen des brevets

(11) Veröffentlichungsnummer : **0 018 533**
**B1**

(12) # EUROPÄISCHE PATENTSCHRIFT

(45) Veröffentlichungstag der Patentschrift : **26.01.83**

(51) Int. Cl.³ : **C 07 C 69/635**, C 07 C 69/65, C 07 C 69/716, C 07 C 69/753

(21) Anmeldenummer : **80102001.7**

(22) Anmeldetag : **14.04.80**

(54) **Substituierte Pentan-(oder Penten-)-carbonsäuren und ihre Derivate, Verfahren zu ihrer Herstellung und ihre Verwendung zur Herstellung von 3-(Aryl-vinyl)-2,2-dimethyl-cyclopropan-1-carbonsäureestern.**

(30) Priorität : **23.04.79 DE 2916417**

(43) Veröffentlichungstag der Anmeldung : **12.11.80 Patentblatt 80/23**

(45) Bekanntmachung des Hinweises auf die Patenterteilung : **26.01.83 Patentblatt 83/04**

(84) Benannte Vertragsstaaten : **BE CH DE FR GB IT LI NL**

(56) Entgegenhaltungen :
**DE A 2 539 895**
**DE A 2 706 184**
**DE A 2 738 150**
**DE A 2 740 849**
**DE A 2 750 182**
**DE A 2 800 073**
**DE A 2 827 101**

(73) Patentinhaber : **BAYER AG**
**Zentralbereich Patente, Marken und Lizenzen**
**D-5090 Leverkusen 1, Bayerwerk (DE)**

(72) Erfinder : **Jautelat, Manfred, Dr.**
**Muellersbaum 28**
**D-5093 Burscheid 1 (DE)**
Erfinder : **Arlt, Dieter, Dr.**
**Rybniker Strasse 2**
**D-5000 Köln 80 (DE)**
Erfinder : **Lantzsch, Reinhard, Dr.**
**Heymannstrasse 32**
**D-5090 Leverkusen 1 (DE)**
Erfinder : **Fuchs, Rainer, Dr.**
**Roeberstrasse 8**
**D-5600 Wuppertal (DE)**
Erfinder : **Riebel, Hans-Jochem, Dr.**
**In der Beek 92**
**D-5600 Wuppertal 1 (DE)**
Erfinder : **Schröder, Rolf, Dr.**
**Pahlkestrasse 17**
**D-5600 Wuppertal 1 (DE)**
Erfinder : **Harnisch, Horst, Dr.**
**Heinenbusch 4**
**D-5203 Much (DE)**

**EP 0 018 533 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

Substituierte Pentan oder (Penten-)-carbonsäuren und ihre Derivate, Verfahren zu ihrer Herstellung und ihre Verwendung zur Herstellung von 3-(Aryl-vinyl)-2,2-dimethyl-cyclopropan-1-carbonsäureestern

Die vorliegende Erfindung betrifft substituierte Pentan oder (Penten-)-carbonsäuren, ihre Derivate, Verfahren zu ihrer Herstellung sowie ihre Verwendung zur Herstellung von Cyclopropancarbonsäureestern.

Es ist bereits bekannt geworden, daß Ester von 3-Styryl-2,2-dimethylcyclopropancarbonsäuren insektizide Eigenschaften besitzen (Deutsche Offenlegunsschrift 2 738 150). Sie werden hergestellt, indem die Knüpfung der C-C-Doppelbindung der Styrylgruppe durch eine Wittigreaktion bei der Butyllithium als Base verwendet wird und unter Schutzgas bei – 78 °C gearbeitet werden muß erfolgt. Dieser Syntheseweg ist für eine technische Herstellung nicht gangbar.

Ferner ist der als Ausgangsprodukt für diese Reaktion vervendete 2,2-Dimethyl-3-formyl-1-carbonsäureester technisch nur schwer zugänglich.

Zur Herstellung dieser Verbindungen sind jedoch substituierte Pentancarbonsäuren und ihre Derivate als preiswerte Ausgangsprodukte gut geeignet.

Es wurden gefunden

1. Verbindungen der Formel

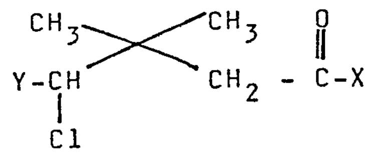

in welcher

Y für die Reste der Formeln

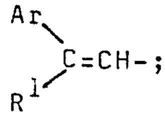
①

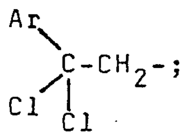
②

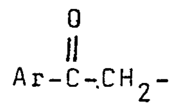
③

steht und

X für Cl oder —O—R steht für den Fall, daß Y für die Reste ① oder ② steht und für —O—R steht für den Fall, daß Y für den Rest ③ steht und

R für Alkyl oder für gegebenenfalls durch Halogen oder Cyano substituiertes 3-Phenoxy-benzyl steht und

$R^1$ für Wasserstoff, Fluor oder Chlor steht, und

Ar für gegebenenfalls durch Halogen, Cyano, Nitro, $C_{1-4}$-Alkyl, $C_{1-4}$-Alkoxy, $C_{1-4}$-Alkylthio, $C_{1-2}$-Halogenalkyl, $C_{1-4}$-Halogenalkoxy, $C_{1-4}$-Halogenalkylthio, gegebenenfalls halogensubstituiertes Alkylsulfonyl, Dialkylamino mit 1 oder 2 Kohlenstoffatomen in jedem

Alkylsubstituenten oder gegebenenfalls halogensubstituiertes Methylendioxy oder Ethylendioxy substituiertes Phenyl steht.

2. Verfahren zur Herstellung von Verbindungen der Formel

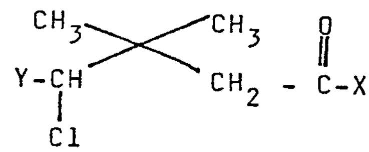

in welcher

Y für die Reste der Formeln

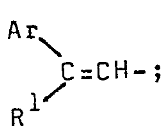

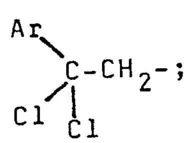

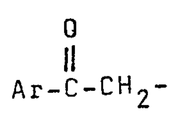

steht und

X für Cl oder —O—R steht für den Fall, daß Y für die Reste ① oder ② steht und für —O—R steht für den Fall, daß Y für den Rest ③ steht und

$R^1$ für Wasserstoff, Fluor oder Chlor steht und

Ar für gegebenenfalls durch Halogen, Cyano, Nitro, $C_{1-4}$-Alkyl, $C_{1-4}$-Alkoxy, $C_{1-4}$-Alkylthio, $C_{1-2}$-Halogenalkyl, $C_{1-4}$-Halogenalkoxy, $C_{1-4}$-Halogenalkylthio, gegebenenfalls halogensubstituiertes Alkylsulfonyl, Dialkylamino mit 1 oder 2 Kohlenstoffatomen in jedem Alkylsubstituenten oder gegebenenfalls halogensubstituiertes Methylendioxy oder Ethylendioxy substituiertes Phenyl steht, dadurch gekennzeichnet, daß man

a) für den Fall, daß Y für den Rest

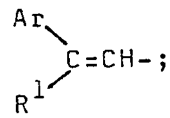

steht, worin

Ar und R die oben angegebene Bedeutung haben,

$a_1$) aus Verbindungen der obigen Formel, in denen Y für den Rest

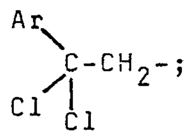

steht, worin

Ar die oben angegebene Bedeutung hat, thermisch Chlorwasserstoff abspaltet oder

$a_2$) Verbindungen der obigen Formel, in denen Y für den Rest

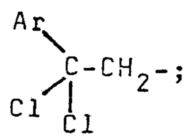

steht, worin

Ar die oben angegebene Bedeutung hat und X für Cl steht, mit Alkoholen der Formel

R—O—H

worin

R die oben angegebene Bedeutung hat,

umsetzt, oder

$a_3$) Verbindungen der Formel (VI)

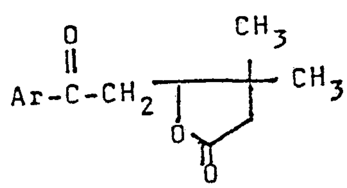

(VI)

worin

Ar die oben angegebene Bedeutung hat, mit mindestens 2 Äquivalent Phosphorpentachlorid umsetzt und anschließend mit einem Alkohol der Formel

R—OH

worin

R die oben angegebene Bedeutung hat, umsetzt oder

$a_4$) Verbindungen der Formel (VI) (oben) reduziert, Wasser abspaltet und mit einem Chlorierungsmittel in Gegenwart eines Alkohols der Formel

R—O—H

worin

R die oben angegebene Bedeutung hat, umsetzt, oder aber nacheinander zunächst den Lactonring der Verbindungen der Formel (VI) mit einem Chlorierungsmittel öffnet und anschließend mit einem Alkohol umsetzt oder

$a_5$) Verbindungen der Formel (V)

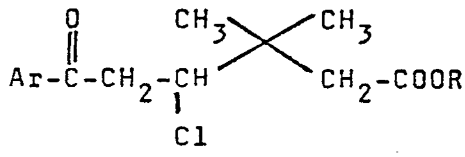 (V)

worin

Ar und R die oben angegebene Bedeutung haben, mit Schwefeltetrafluorid umsetzt, oder

$a_6$) Verbindungen, in denen Y für die Reste

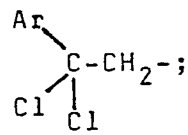

oder

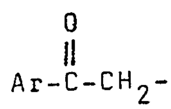

worin

Ar die oben angegebene Bedeutung hat, steht, mit wasserfreiem Fluorwasserstoff umsetzt und gegebenenfalls mit einem Alkohol der Formel

R—O—H

worin

R die oben angegebene Bedeutung hat, umsetzt, sowie

b) für den Fall, daß Y für den Rest

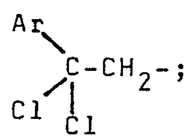

worin

Ar die oben angegebene Bedeutung hat, steht und X für —O—R steht, (wobei R die oben angegebene Bedeutung hat), Verbindungen, in denen Y für den Rest

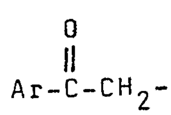

worin

Ar die oben angegebene Bedeutung hat, steht, mit Phosphorpentachlorid in einem Verdünnungsmittel unterhalb 30 °C umsetzt.

3. Verwendung von Verbindungen der Formel

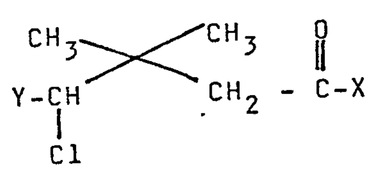

in welcher
X für Cl oder —O—R steht und
Y für die Reste der Formeln

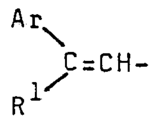;
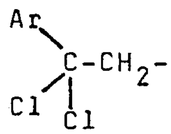;
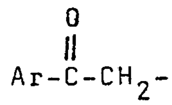

steht und
R für Alkyl oder für gegebenenfalls durch Halogen oder Cyano substituiertes 3-Phenoxy-benzyl steht und
Ar für gegebenenfalls durch Halogen, Cyano, Nitro, $C_{1-4}$-Alkyl, $C_{1-4}$-Alkoxy, $C_{1-4}$-Alkylthio, $C_{1-2}$-Halogenalkyl, $C_{1-4}$-Halogenalkoxy, $C_{1-4}$-Halogenalkylthio, gegebenenfalls halogensubstituiertes Alkylsulfonyl, Dialkylamino mit 1 oder 2 Kohlenstoffatomen in jedem Alkylsubstituenten oder gegebenenfalls halogensubstituiertes Methylendioxy oder Ethylendioxy substituiertes Phenyl steht,
$R^1$ für Wasserstoff, Fluor oder Chlor steht, zur Herstellung von Cyclopropan-1-carbonsäureestern der allgemeinen Formel (I)

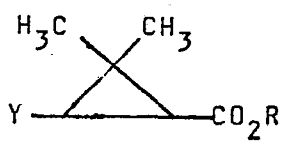
(I)

in der

Y für

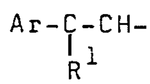

oder

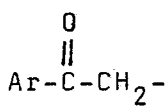

steht, und
Ar für gegebenenfalls durch Halogen, Cyano, Nitro, $C_{1-4}$-Alkyl, $C_{1-4}$-Alkoxy, $C_{1-4}$-Alkylthio, $C_{1-2}$-Halogenalkyl, $C_{1-4}$-Halogenalkoxy, $C_{1-4}$-Halogenalkylthio, gegebenenfalls halogensubstituiertes Alkylsulfonyl, Dialkylamino mit 1 oder 2 Kohlenstoffatomen in jedem Alkylsubstituenten oder gegebenenfalls halogensubstituiertes Methylendioxy oder Ethylendioxy substituiertes Phenyl steht,
R für Alkyl oder für gegebenenfalls durch Halogen oder Cyano substituiertes 3-Phenoxybenzyl steht und
$R^1$ für Wasserstoff, Fluor oder Chlor steht, dadurch gekennzeichnet, daß man Verbindungen der Formel

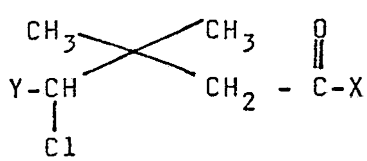

in welcher
Y und X die oben angegebene Bedeutung haben, mit einer äquivalenten Menge einer Base, gegebenenfalls in Gegenwart eines Verdünnungsmittels zwischen −20 und +60 °C umsetzt und gegebenenfalls mit einem Alkohol der Formel R—O—H umsetzt.
4. Verbindungen der Formel (II)

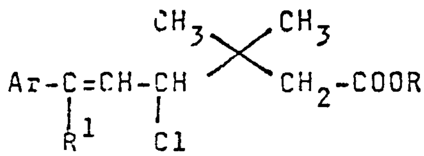

(II)

in welcher

Ar, R und $R^1$ die in Anspruch 1 angegebene Bedeutung haben.

5. Verbindungen der Formel (III)

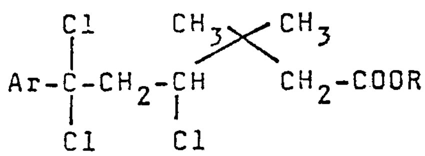

(III)

in welcher

Ar und R die in Anspruch 1 angegebene Bedeutung haben.

6. Verbindungen der Formel (IV)

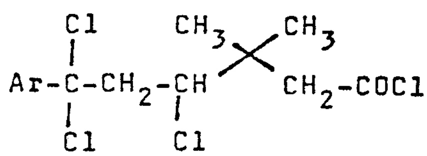

(IV)

in welcher

Ar die in Anspruch 1 angegebene Bedeutung hat.

7. Verbindungen der allgemeinen Formel (IV) werden hergestellt, indem man Verbindungen der allgemeinen Formel (VI)

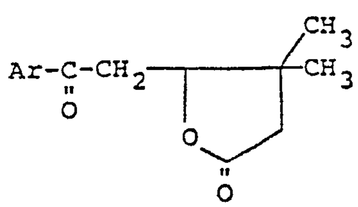

(VI)

in welcher

Ar die oben angegebene Bedeutung hat, mit mindestens zwei Äquivalenten Phosphorpentachlorid in einem Verdünnungsmittel unterhalb 30 °C umsetzt.

8. Verbindungen der allgemeinen Formel (V) werden hergestellt, indem man Verbindungen der Formel (VI) mit einem Chlorierungsmittel in Gegenwart eines Alkohols R—OH umsetzt, wobei R die unter 1. (oben) angegebene Bedeutung besitzt oder aber nacheinander zunächst den Lactonring mit einem Chlorierungsmittel öffnet und anschließend mit dem Alkohol umsetzt.

9. Verbindungen der allgemeinen Formel (VI) werden hergestellt, indem man Verbindungen der Formel (VII)

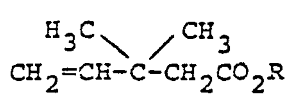

(VII)

in der

R $C_1$-$C_4$-Alkyl bedeutet mit Verbindungen der Formel (VIII)

Ar—CO—Hal (VIII)

in welcher

Ar die unter 1. (oben) angegebene Bedeutung besitzt und

Hal Halogen, vorzugsweise Chlor bedeutet, in Gegenwart eines Friedel-Crafts-Katalysators umsetzt.

10. Verbindungen der allgemeinen Formel (VI), die sich unter der allgemeinen Formel (IX) zusammenfassen lassen

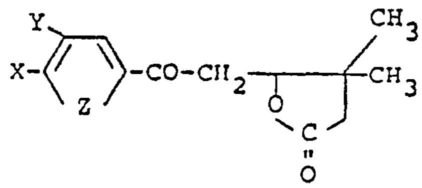

(IX)

in welcher

X für Wasserstoff, $C_{1-4}$-Alkyl, Halogen, Aryl, Aralkyl, Aryloxy, Arylthio, $C_{1-4}$-Alkoxy oder $C_{1-4}$-Alkylthio steht,

Y für Wasserstoff steht oder gleich X ist, oder

X und Y für Methylendioxy oder Äthylendioxy stehen und

Z für O, S oder —CH = CH— steht, werden erhalten, indem man Verbindungen der allgemeinen Formel (X)

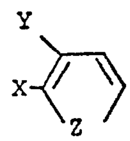

(X)

wobei

X, Y und Z die oben angegebene Bedeutung besitzen, mit dem Säurechlorid der Formel (XI)

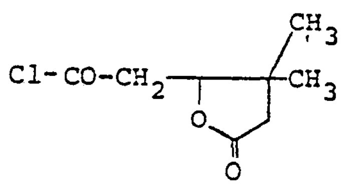

(XI)

in Gegenwart eines Friedel-Crafts-Katalysators sowie gegebenenfalls in Gegenwart eines Verdünnungsmittels umsetzt.

11. Die Verbindung der Formel (XI) wird hergestellt, indem die Verbindung der Formel (XII)

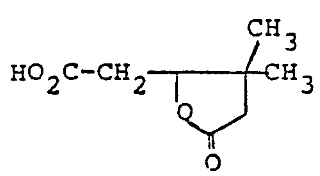

(XII)

mit einem Chlorierungsmittel umgesetzt wird.

Die erfindungsgemäßen Verfahren unter Verwendung der neuen Verbindungen dienen zur Herstellung unter anderem der aus DE-OS 2 738 150 bekannten Verbindungen. Sie sind einfacher und technisch leichter durchführbar als das aus DE-OS 2 738 150 bekannte Verfahren zur Herstellung dieser Verbindungen.

(Auf die Anmeldung EP 80101997.7 der Anmelderin vom selben Tag, sei hingewiesen).

Verwendet man beim Verfahren 3 6-(4'-Chlor)-phenyl-4,6-dichlor-3,3-dimethyl-hex-5-en-säureäthylester als Ausgangstoff, kann der Reaktionsablauf durch folgendes Formelschema wiedergegeben werden.

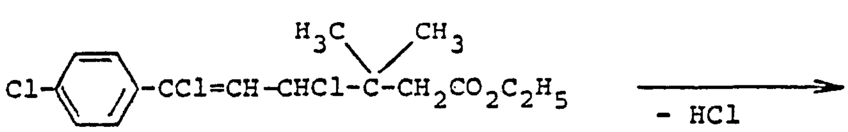

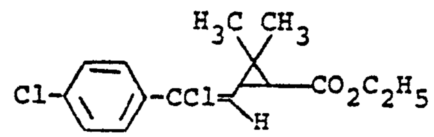

Die beim Verfahren 3 verwendbaren Ausgangsstoffe sind durch die allgemeine Formel

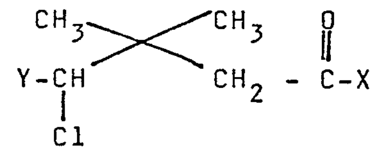

definiert. Darin steht im Rest Y der Rest Ar besonders bevorzugt für einen durch einen oder zwei gleiche oder verschiedene Substituenten X substituierten Phenylring, wobei X bevorzugt Wasserstoff, Fluor, Chlor, Brom, Cyano, Nitro, Methyl, Äthyl, Isopropyl, tert.-Butyl, Methoxy, Äthoxy, Methylmercapto, Trifluormethyl, Methylsuflonyl, Trifluormethylsulfonyl, Dimethylamino, Diäthylamino, gegebenenfalls halogensubstituiertes Methylendioxy oder Äthylendioxy bedeutet, R bevorzugt für $C_{1-4}$-Alkyl oder für einen gegebenenfalls durch Halogen oder Cyano substituierten 3-Phenoxybenzylrest und $R^1$ für Wasserstoff, Fluor oder Chlor.

Ganz besonders bevorzugt steht Ar für einen durch ein oder zwei gleiche oder verschiedene Substituenten X in 3- und/oder 4-Stellung substituierten Phenylring, wobei X bevorzugt Wasserstoff, Fluor, Chlor, Brom, Methyl, Methoxy, Äthoxy, Trifluormethyl, Nitro, Methylendioxy oder Äthylendioxy bedeutet, ganz besonders bevorzugt steht R für Methyl oder Ethyl und $R^1$ für Chlor.

Verwendet man beim Verfahren 3 6-(4'-Fluorphenyl)-4,6,6-trichlor-3,3-dimethyl-hexansäuremethylester als Ausgangsstoff, kann der Reaktionsablauf durch folgendes Formelschema wiedergegeben werden :

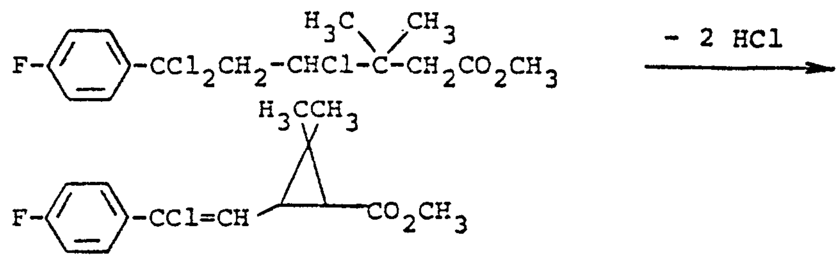


Verwendet man beim Verfahren 3 6-(4'-Methyl)-phenyl-4,6,6-trichlor-3,3-dimethyl-hexansäurechlorid als Ausgangsstoff, kann der Reaktionsverlauf durch folgendes Formelschema wiedergegeben werden :

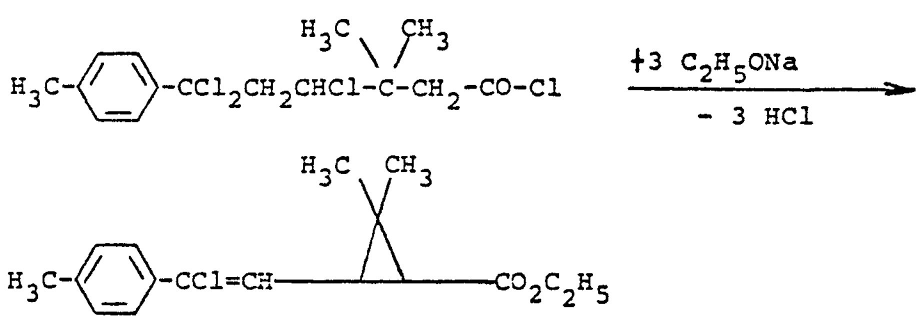


Verfahren 3 wird durchgeführt, indem man die Ausgangsstoffe mit 1,2 bzw. 3 Äquivalenten Base gegebenenfalls in Gegenwart eines Verdünnungsmittels umsetzt.

Als Basen finden bevorzugt Alkoholate Verwendung, wie Natriummethylat, Kaliumäthylat, Natriumäthylat, Natriumisopropylat, Natrium-tert.-butylat oder Kalium-tert.-butylat.

Als Verdünnungsmittel wird bevorzugt der der Base entsprechende Alkohol eingesetzt, es können jedoch auch zusätzlich oder ausschließlich andere inerte Verdünnungsmittel verwendet werden, wie beispielsweise Kohlenwasserstoffe, wie Toluol, Xylol, Cyclohexan oder Chlorkohlenwasserstoffe, wie Chlorbenzol oder Äther, wie Diisopropyläther, THF oder Dioxan.

Das Verfahren wird bei Temperaturen zwischen − 20 °C und + 60 °C, vorzugsweise zwischen 20 °C

und 50 °C durchgeführt.

Eine ähnliche Reaktion ist in der DE-OS 2 539 896 beschrieben. Doch während dort der bevorzugte Temperaturbereich — bei Anwendung von Natriummethylat oder Natriumäthylat — zwischen 60 und 100 °C liegt (Seite 31), wird unter diesen Bedingungen nur sehr wenig Produkt der Formel (I) erhalten, sondern hauptsächlich ein weiteres Mol Halogenwasserstoff unter Bildung einer Dreifachbindung abgespalten.

Überraschend wurde gefunden, daß dies vermieden wird, wenn unterhalb von 60 °C gearbeitet wird.

Ein weiterer Vorteil des erfindungsgemäßen Verfahrens ist, daß ganz bevorzugt nur eines der 4 möglichen Stereoisomeren gebildet wird. Es besitzt die trans-Figuration bezogen auf den Cyclopropanring.

Die insektiziden und akariziden Ester dieses Isomeren zeigen eine besonders gute Wirksamkeit.

Nach Verfahren 3 lassen sich bevorzugt folgende Cyclopropancarbonsäureester der Formel (I) herstellen.

2,2-Dimethyl-3-[2'-chlor-2'-(4'-fluor-phenyl)-vinyl]-cyclopropan-1-carbonsäuremethyl und -äthylester

2,2-Dimethyl-3-[2'-chlor-2'-(4'-chlor-phenyl)-vinyl]-cyclopropan-1-carbonsäuremethyl und -äthylester

2,2-Dimethyl-3-[2'-chlor-2'-(4'-brom-phenyl)-vinyl]-cyclopropan-1-carbonsäuremethyl- und äthylester

2,2-Dimethyl-3-[2'-chlor-2'-phenyl-vinyl]-cyclopropan-1-carbonsäuremethyl- und äthylester

2,2-Dimethyl-3-[2'-chlor-2'-(3'-chlor-phenyl)-vinyl]-cyclopropan-1-carbonsäuremethyl- und äthylester

2,2-Dimethyl-3-[2'-(phenyl)-vinyl]-cyclopropan-1-carbonsäuremethyl- und äthylester

2,2-Dimethyl-3-[2'-(4'-chlor-phenyl)-vinyl]-cyclopropan-1-carbonsäuremethyl- und äthylester

2,2-Dimethyl-3-[2'-chlor-2'-(3',4'-dichlor)-phenyl-vinyl]-cyclopropan-1-carbonsäuremethyl- und äthylester

2,2-Dimethyl-3-[2'-chlor-2'-(4'-methyl-phenyl)-vinyl]-cyclopropan-1-carbonsäuremethyl- und äthylester

2,2-Dimethyl-3-[2'-chlor-2'-(3'-methyl-phenyl)-vinyl]-cyclopropan-1-carbonsäuremethyl- und äthylester

2,2-Dimethyl-3-[2'-chlor-2'-3'-methyl-4'-chlor-phenyl)-vinyl]-cyclopropan-1-carbonsäuremethyl- und äthylester.

Die Verbindungen der allgemeinen Formel

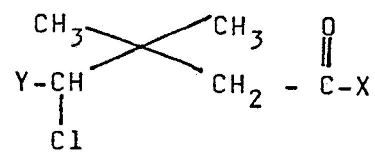

sind neu.

Sie werden nach dem unter 2a (oben) angegebenen Verfahren erhalten.

Verwendet man bei Verfahren $2a_1$ 6-(4'-Chlor)-phenyl-4,6,6-trichlor-3,3-dimethyl-hexansäureäthylester als Ausgangsstoff, kann der Reaktionsablauf durch folgendes Formelschema wiedergegeben werden :

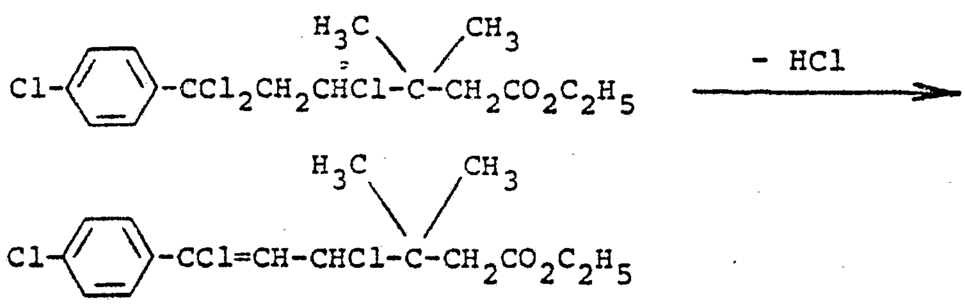


Die beim Verfahren $2a_1$ verwendbaren Ausgangsstoffe sind durch die allgemeine Formel (III) definiert. Die bevorzugten und besonders bevorzugten Substituenten Ar und R sind die gleichen wie bei Verfahren 3.

Die Verbindungen der Formel (III) sind neu ; ihre Herstellung wird weiter unten beschrieben.

Verfahren $2a_1$ wird durchgeführt, indem man die Ausgangsstoffe der Formel (III), gegebenenfalls in einem Verdünnungsmittel, auf Temperaturen zwischen 25 und 80 °C, vorzugsweise auf Temperaturen zwischen 30 und 50 °C, erwärmt. Hierbei spaltet sich Chlorwasserstoff ab. Als Verdünnungsmittel

kommen insbesondere Kohlenwasserstoffe wie Benzol, Toluol, Xylol, Benzin, Cyclohexan, Petroläther in Frage, aber auch Chlorkohlenwasserstoffe wie Methylenchlorid, Chloroform, Tetrachlorkohlenstoff, Dichloräthan oder Chlorbenzol, sowie Nitrile, wie Acetonitril, in Frage.

Gegebenenfalls kann auf die Isolierung der Verbindungen der allgemeinen Formel (III) verzichtet werden.

Verwendet man bei Verfahren $2a_2$ 6-(4'-Chlor)-phenyl-4,6,6-trichlor-3,3-dimethylhexansäurechlorid als Ausgangsstoff, kann der Reaktionsverlauf durch folgendes Formelschema wiedergegeben werden :

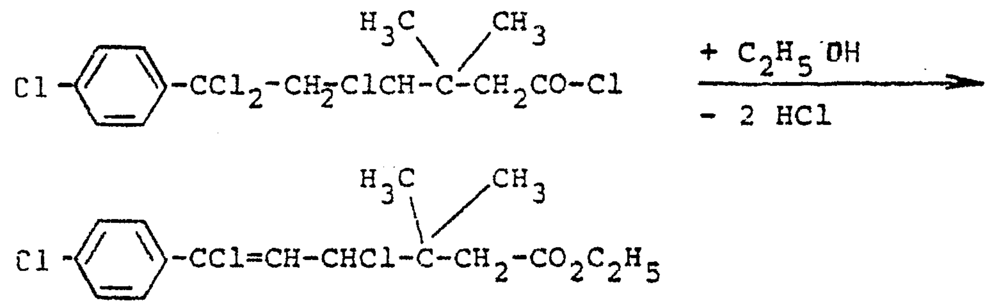


Die beim Verfahren $2a_2$ verwendbaren Ausgangsstoffe sind durch die allgemeine Formel (IV) definiert. Die bevorzugten und besonders bevorzugten Substituenten Ar sind die gleichen wie bei Verfahren 3.

Die Verbindungen der Formel (IV) sind neu ; ihre Herstellung wird weiter unten beschrieben.

Verfahren $2a_2$ wird durchgeführt, indem man die Ausgangsstoffe der Formel (IV) gegebenenfalls in einem Verdünnungsmittel bei Temperaturen zwischen – 20 °C und 80 °C, vorzugsweise zwischen 0 °C und 30 °C mit einem Alkohol der allgemeinen Formel R—OH versetzt, wobei die bevorzugte Bedeutung von R die gleiche ist, wie bei Verfahren 3. Zur Beendigung der Reaktion wird anschließend noch eine Zeitlang bei erhöhter Temperatur, vorzugsweise zwischen 30 und 60 °C gerührt. Als Verdünnungsmittel kann überschüssiger Alkohol verwendet werden, sowie alle Lösungsmittel, die auch in Verfahren $2a_1$ Verwendung finden können.

Verwendet man bei Verfahren $2a_3$ 3,3-Dimethyl-4-(4'-chlor)-phenacyl-8-butyrolacton und Äthanol als Ausgangsstoffe, kann der Reaktionsablauf durch folgendes Formelschema wiedergegeben werden :

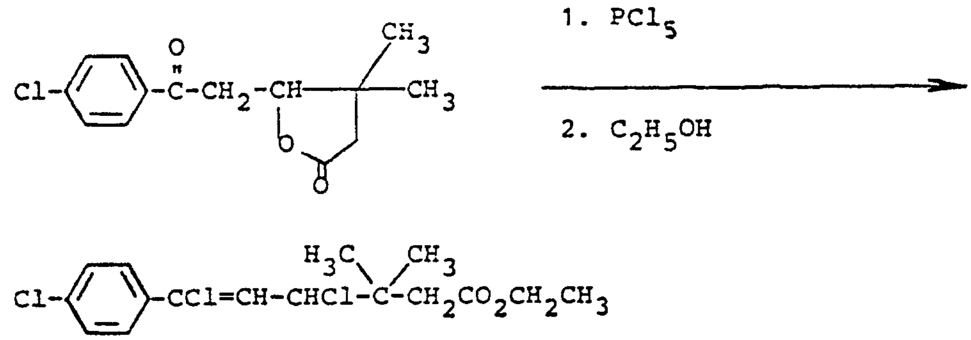


Die beim Verfahren $2a_3$ verwendbaren Ausgangsstoffe sind durch die allgemeine Formel (VI) definiert. Die bevorzugten und besonders bevorzugten Substituenten Ar sind die gleichen wie bei Verfahren 3. Die Verbindungen der Formel (VI) sind neu ; ihre Herstellung wird weiter unten beschrieben.

Als Chlorierungsmittel kann Phosphorpentachlorid verwendet werden.

Verfahren $2a_3$ wird in der Weise durchgeführt, daß man die Ausgangsstoffe der allgemeinen Formel (VI) vorlegt und mit mindestens zwei Äquivalenten des Chlorierungsmittels umsetzt. Ein geringer Überschuß ist günstig.

Vorzugsweise wird — im Gegensatz zur allgemein üblichen Arbeitsweise (Houben-Weyl, Band V, 3, Seite 912 ff) bei der Umsetzung von ketonen mit Phosphorpentachlorid — in Gegenwart eines Verdünnungsmittels gearbeitet. Als Verdünnungsmittel kommen die gleichen wie die bei Verfahren $2a_1$ genannten in Frage.

Durch Anwendung eines Verdünnungsmittels sowie Zudosieren des Phosphorpentachlorids können überraschenderweise die sonst üblichen Nebenreaktionen, wie beispielsweise eine α-Chlorierung zur Carbonylgruppe oder eine Addition von Chlorwasserstoff an die Chlorvinylgruppe vermieden werden.

Die Umsetzungstemperatur liegt zwischen – 20 °C und + 60 °C, vorzugsweise zwischen 0 °C und 35 °C.

Die anschließende Veresterung erfolgt durch Zutropfen von überschüssigem Alkohol R—OH analog Verfahren $2a_2$.

Die Aufarbeitung erfolgt durch Neutralwaschen und Abtrennen der organischen Phase, sowie

Abdestillieren des Lösungsmittels und des Phosphorsäureesters. Auf eine Reinigung durch Destillation kann auf diese Stufe meist verzichtet werden.

Verwendet man bei Verfahren $2a_4$ 3,3-Dimethyl-4-phenacyl-γ-butylrolacton als Ausgangsstoff, kann der Reaktionsverlauf durch folgendes Formelschema wiedergegeben werden :

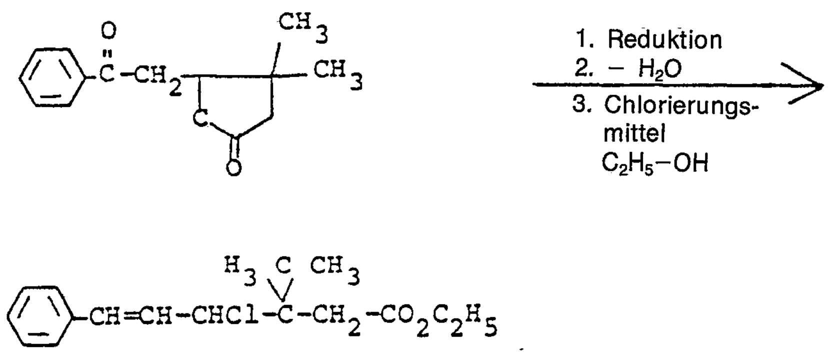


Die beim Verfahren $2a_4$ verwendbaren Ausgangsstoffe sind durch die allgemeine Formel (VI) definiert. Die bevorzugten und besonders bevorzugten Substituenten Ar sind die gleichen wie bei Verfahren 3. Die Verbindungen der Formel (VI) sind neu ; ihre Herstellung wird weiter unten beschrieben.

Als Reduktionsmittel kommen grundsätzlich alle Mittel in Frage, durch die ein Keton zum Alkohol reduziert wird, ohne den Lactonring anzugreifen. Beispielsweise seien genannt : komplexe Borhydride, wie Natriumborhydrid oder Wasserstoff in Gegenwart von beispielsweise Nickel-, Palladium- oder Platin-Katalysatoren, wie beispielsweise Raney-Nickel. Natriumborhydrid ist bevorzugt.

Verfahren $2a_4$ wird in der Weise durchgeführt, daß man nach dem Reduktionsschritt, der in an sich üblicher Weise durchgeführt wird, dehydratisiert, d.h. Wasser abspaltet.

Zur Dehydratisierung werden bevorzugt saure Katalysatoren eingesetzt. Beispielsweise seien genannt : Oxalsäure, Schwefelsäure, Phosphorsäure, Kaliumhydrogensulfat, p-Toluolsulfonsäure, Aluminiumoxid, Silikate. Außerdem kann der entstandene Alkohol acetyliert und anschließend durch Erhitzen Essigsäure abgespalten werden. Die Acetylierung erfolgt mit Acetylchlorid oder Acetanhydrid. Der dritte Teilschritt des Verfahrens $2a_4$ entspricht Verfahren 9 (unten).

Verwendet man bei Verfahren $2a_5$ 6-(4'-Chlor)-phenyl-6-oxo-4-chlor-3,3-dimethyl-hexansäureäthylester als Ausgangsstoff, so kann der Reaktionsablauf durch folgendes Formelschema wiedergegeben werden

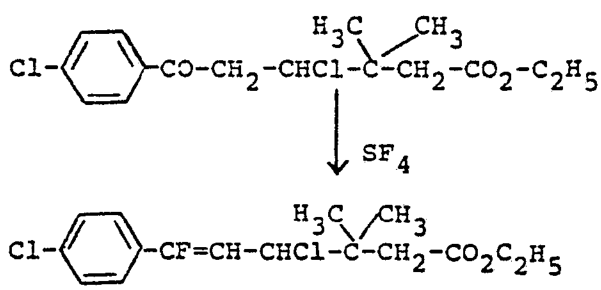


Die beim Verfahren $2a_5$ verwendbaren Ausgangsstoffe sind durch die Formel (V) definiert. Die bevorzugten und besonders bevorzugten Substituenten Ar und R sind die gleichen wie bei Verfahren 3. Die Verbindungen der Formel (V) sind neu ; ihre Herstellung wird weiter unten beschrieben.

Verfahren $2a_5$ wird in der Weise durchgeführt, daß man die Ausgangsstoffe der Formel (V) zusammen mit Schwefeltetrafluorid in einem Autoklaven auf Temperaturen zwischen 100 und 180 °C, vorzugsweise auf 120-150 °C erhitzt werden. Als Katalysator können gegebenenfalls HF, $BF_3$, $TiF_4$ oder ähnliche Verbindungen verwendet werden. Die Aufarbeitung erfolt in üblicher Weise ; die Rohprodukte werden direkt nach Verfahren 3 weiter umgesetzt.

Verwendet man bei Verfahren $2a_6$ 6-Phenyl-4,6,6-trichlor-3,3-dimethyl-hexansäureethylester als Ausgangstoffe, kann der Reaktionsablauf durch das folgende Formelschema wiedergegeben werden :

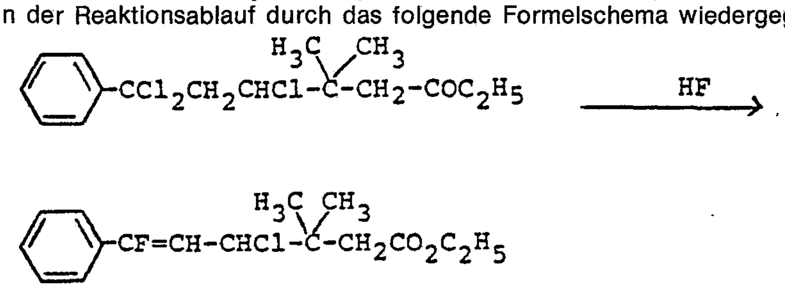

Verwendet man bei Verfahren $2a_6$ 6-Phenyl-4,6,6-trichlor-3,3-dimethyl-hexansäurechlorid als Ausgangsstoff, so kann der Reaktionsablauf durch das folgende Formelschema wiedergegeben werden :

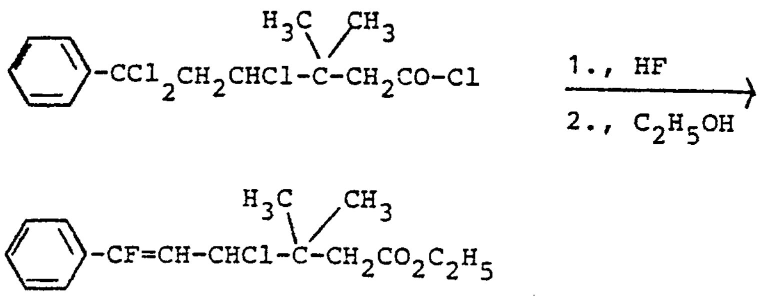


Die beim Verfahren $2a_6$ verwendbaren Ausgangsstoffe sind durch die allgemeinen Formeln III bzw. IV definiert.

Die bevorzugten und besonders bevorzugten Substituenten Ar und R sind die gleichen wie bei Verfahren 3. Die Verbindungen der Formel (III) bzw. (IV) sind neu ; ihre Herstellung wird weiter unten beschrieben.

Die Umsetzung kann bei dem erfindungsgemäßen Verfahren bei Temperaturen von – 20 °C bis 80 °C erfolgen, besonders bevorzugt sind Temperaturen von 0 ° bis 50 °C.

Die Flußsäure wird im allgemeinen im Überschuß eingesetzt. Die Reaktion kann in Gegenwart von Lösungsmitteln, wie auch ohne solche ausgeführt werden. Als Lösungsmittel kommen ganz allgemein inerte aprotische Flüssigkeiten in Frage. Bevorzugt sind beispielsweise Methylenchlorid, Trichlorfluormethan oder Tetrachlorkohlenstoff.

Das erfindungsgemäße Verfahren kann wie folgt durchgeführt werden :

Im Reaktionsgefäß wird bei ca. – 20 °C die wasserfreie Flußsäure vorgelegt und unter Rühren die Ausgangsstoffe der Formel III. bzw. IV in Methylenchlorid gelöst zugetropft. Bereits bei 0 °C entwickelt sich heftig Chlorwasserstoff. Nach beendeter Chlorwasserstoff-Entwicklung kann die Temperatur auf Raumtemperatur oder mehr erhöht werden. Anschließend wird die überschüssige Flußsäure unter Normaldruck oder vermindertem Druck, sowie das Lösungsmittel, abdestilliert. Der Rückstand wird, falls Ausgangsstoffe der Formel IV verwendet wurden, mit Alkoholen der Formel R—OH zum Ester umgesetzt und anschließend direkt in Verfahren 3 eingesetzt.

Verwendet man bei Verfahren 2b 6-(3'-Chlor)-phenyl-6-oxo-4-chlor-3,3-dimethyl-hexansäureäthylester als Ausgangsstoff, so kann der Reaktionsablauf durch folgendes Formelschema wiedergegeben werden :

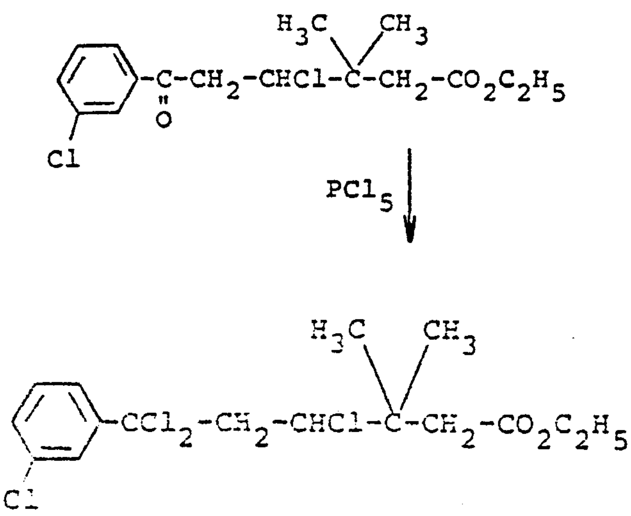


Die beim Verfahren 2b verwendbaren Ausgangsstoffe sind durch die allgemeine Formel (V) definiert.

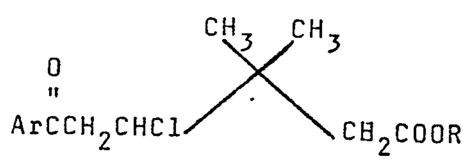

(V)

in welcher

Ar und R die unter 1 angegebene Bedeutung besitzen.

Die bevorzugten und besonders bevorzugten Substituenten Ar und R sind die gleichen wie bei Verfahren 3. Die Verbindungen der Formel (V) sind neu ; ihre Herstellung wird weiter unten beschrieben.

Es wurde gefunden, daß nur dann die Verbindungen (III) aus den Verbindungen der Formel (V) erhalten werden, wenn man in einem Verdünnungsmittel unterhalb von 30 °C arbeitet.

Verfahren 2b wird in der Weise durchgeführt, daß man die Ausgangsstoffe der allgemeinen Formel (III) in einem Verdünnungsmittel löst. Als Verdünnungsmittel kommen die gleichen wie die bei Verfahren $2a_1$ genannten in Frage, bevorzugt sind Petroläther, Cyclohexan, Toluol und Chlorbenzol.

Die Umsetzungstemperatur liegt zwischen – 20 °C und 30 °C, vorzugsweise zwischen 0 und 25 °C.

Die Aufarbeitung erfolgt durch Versetzen mit Eiswasser, neutral waschen der organischen Phase und Abdestillieren des Lösungsmittels.

Der Konstitutionsbeweis erfolgt durch ein Kernresonanzspektrum.

Verwendet man bei Verfahren 7) 3,3-Dimethyl-4-(4'-brom)-phenacyl-γ-butyrolacton als Ausgangsstoff, kann der Reaktionsverlauf durch folgendes Formelschema wiedergegeben werden :

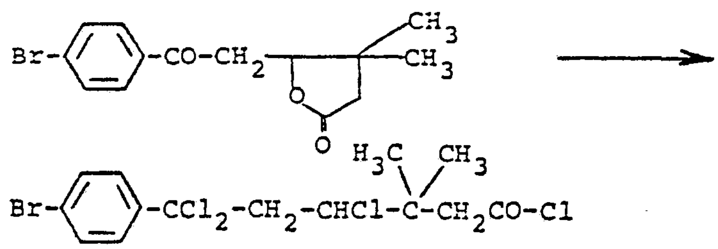

Die beim Verfahren 7 verwendbaren Ausgangsstoffe sind durch die allgemeine Formel (VI) definiert. Die bevorzugten Substituenten sind die gleichen wie bei Verfahren 3. Die Verbindungen der Formel (VI) sind neu ; ihre Herstellung wird weiter unten beschrieben.

Es wurde weiter gefunden, daß nur dann die Verbindungen (IV) aus den Verbindungen (VI) erhalten werden, wenn man in einem Verdünnungsmittel unterhalb von 30 °C arbeitet.

Verfahren 7 wird in der Weise durchgeführt, daß man die Ausgangsstoffe der allgemeinen Formel (VI) in einem Verdünnungsmittel löst.

Als Verdünnungsmittel kommen die gleichen wie die bei Verfahren 2a genannten in Frage, bevorzugt sind Petroläther, Cyclohexan, Toluol und Chlorbenzol.

Die Umsetzungstemperatur liegt zwischen – 20° und + 30 °C, vorzugsweise zwischen 0 und 25 °C.

Die Verbindungen der Formel (IV) können durch schonendes Abdestillieren von Lösungsmittel und Phosphoroxychlorid isoliert werden oder direkt nach Verfahren 1c oder 6b weiter umgesetzt werden.

Verwendet man bei Verfahren 8) 3,3-Dimethyl-4-(4'-fluor)-phenacyl-γ-butyrolacton und Äthanol als Ausgangsstoffe, kann der Reaktionsverlauf durch folgendes Formelschema wiedergegeben werden :

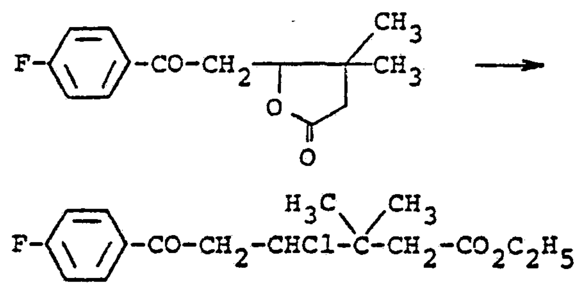

Die beim Verfahren 8 verwendbaren Ausgangsstoffe sind durch die allgemeine Formel (VI) definiert. Die bevorzugten und besonders bevorzugten Substituenten Ar sind die gleichen wie bei Verfahren 1a. Die Verbindungen der Formel (VI) sind neu ; ihre Herstellung wird weiter unten beschrieben.

Der zweite Ausgangsstoff für Verfahren 8 ist ein Alkohol der Formel R—OH, wobei die bevorzugte Bedeutung von R die gleiche wie die Verfahren $2a_2$ ist.

Folgende Chlorierungsmittel können bei Verfahren 8 Verwendung finden : Chlorwasserstoff, Thionylchlorid, Phosgen oder Phosphortrichlorid, bevorzugt sind Chlorwasserstoff und Thionylchlorid, gegebenenfalls ist Dimethylformamid als Katalysator zu verwenden.

Grundsätzlich sind Lactonringöffnungen dieser Art bekannt, jedoch enthalten solche Lactone in keinem Fall eine zusätzliche Carbonylgruppe. Es mußten daher Bedingungen gefunden werden, um zu erreichen, daß nur die gewünschten Reaktion abläuft, nicht aber beispielsweise 1) sauer katalysierte Kondensationen des Ketons mit sich selbst (so reagiert beispielsweise Acetophenon unter Chlorwasserstoff-Katalyse mit sich selbst) 2) Chlorierung der Carbonylgruppe oder der Wasserstoffatome in α-Position zur Carbonylgruppe.

Erfindungsgemäß wird Verfahren 8 in der Weise durchgeführt, daß der Ausgangsstoff der Formel (VI) in einem Alkohol der Formel R—OH gelöst wird, gegebenenfalls noch ein Verdünnungsmittel zugesetzt wird und anschließend das Chlorierungsmittel eingeleitet oder zugetropft wird. Es setzt eine exotherme Reaktion ein ; um die oben erwähnten Nebenreaktionen zu vermeiden sollte die Temperatur auf keinen Fall 80 °C überschreiten, vorzugsweise wird jedoch zwischen 20 und 50 °C gearbeitet.

Als Verdünnungsmittel kommen insbesondere Benzol, Toluol, Benzin, Petroläther, Cyclohexan, Chlorbenzol oder Xylol in Frage.

Grundsätzlich ist es auch möglich, zuerst den Lactonring zu öffnen und dann Alkohol zuzusetzen. In diesem Fall erhitzt man die Ausgangsverbindungen (VI) gegebenenfalls in einer der erwähnten Verdünnungsmittel auf Temperaturen zwischen 50 und 80 °C (höhere Temperaturen sind unzweckmäßig) unter Zugabe eines Chlorierungsmittels, gegebenenfalls unter Druck. Anschließend wird der Alkohol R—OH zugetropft oder zugepumpt.

Die Isolierung der Verbindungen der Formel (V) erfolgt durch schonendes Abdestillieren des Lösungsmittels. Eine weitere Reinigung ist schwierig, aber auch nicht notwendig. Die rohen Verbindungen der Formel (V) können direkt für Verfahren 2b verwendet werden.

Verwendet man bei Verfahren 9) 3,3-Dimethyl-pent-4-en-säuremethylester und Benzoylchlorid als Ausgangsstoffe, kann der Reaktionsablauf durch folgendes Formelschema wiedergegeben werden :

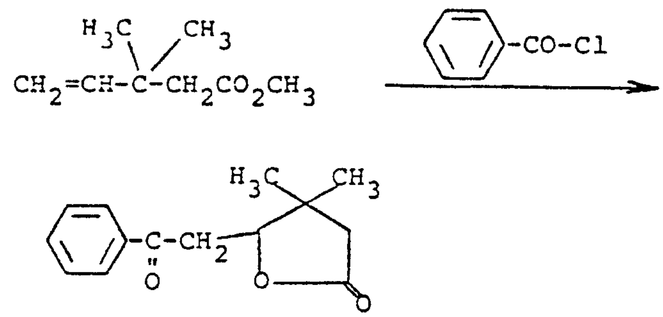

Die beim Verfahren 9 verwendbaren Ausgangsstoffe sind durch die allgemeinen Formeln (VII) und (VIII) definiert. Die bevorzugten und besonders bevorzugten Substituenten Ar sind die gleichen wie bei Verfahren 3. Die Verbindungen der Formel (VII) (DT-OS 2 539 895) und (VIII) sind bekannt.

Beispiele für Verbindungen der Formel (VIII) sind : Benzoylchlorid, 4-Chlorbenzoylchlorid, 3-Chlorbenzoylchlorid, 2-Chlorbenzoylchlorid, 4-Fluorbenzoylchlorid, 4-Brombenzoylchlorid, 3,4-Dichlorbenzoylchlorid, 4-Methyl-benzoylchlorid, 4-Phenoxybenzoylchlorid, 3-Phenoxybenzoylchlorid, 4-Trifluormethyl-benzoylchlorid, 3-Trifluor-methylbenzoylchlorid, 3-Trifluormethyl-4-chlorbenzoylchlorid, Pentachlorbenzoylchlorid, Pentafluorbenzoylchlorid, Pentafluorbenzoylchlorid, 2,4,6-Trifluor-3,5-dichlor-benzoylchlorid, 2,6-Difluor-3,5-dichlorbenzoylchlorid, 4-Nitrobenzoylchlorid, 4-Methylsulfonylbenzoylchlorid.

Als Katalysatoren kommen grundsätzlich alle üblichen Friedel-Crafts-Katalysatoren in Frage. Besonders bevorzugt ist Zinntetrachlorid oder gegebenenfalls Mischungen mit Aluminiumchlorid, Titantetrachlorid, Zinkchlorid oder Eisen (III) chlorid. Mit Aluminiumchlorid allein entstehen nicht die Verbindungen der Formel (VI), sondern der Formel (V). Der Friedel-Crafts-Katalysator wird äquimolar, im Unterschuß oder im Überschuß eingesetzt. Verfahren 9 kann mit oder ohne Verdünnungsmittel durchgeführt werden. Wird ein solches verwendet, so kommen beispielsweise Methylenchlorid, Chloroform, Dichloräthan, Tetrachloräthan, Nitromethan oder Nitrobenzol in Frage.

Das erfindungsgemäße Verfahren wird in der Weise durchgeführt, daß der Friedel-Crafts-Katalysator, gegebenenfalls in einem Verdünnungsmittel, vorgelegt wird und das Säurehalogenid der allgemeinen Formel (VIII) unter Kühlung zugegeben wird. Anschließend wird ein Ester der allgemeinen Formel (VII) bei Temperaturen zwischen − 25 °C bis + 50 °C, vorzugsweise bei Temperaturen zwischen 0 °C und 25 °C zugetropft.

Es ist jedoch auch möglich, ein Säurehalogenid der Formel (VIII) zusammen mit einem Ester der Formel (VII) gegebenenfalls in Gegenwart eines Verdünnungsmittels, vorzulegen und anschließend den Friedel-Crafts-Katalysator zuzudosieren.

Zur Reaktionsbeschleunigung kann nach beendetem Mischen der Reaktionsteilnehmer bei erhöhter Temperatur gearbeitet werden, z.B. bei 25 bis 150 °C, vorzugsweise bei 30 bis 100 °C. Das Ende der Reaktion ist am Ende der Gasentwicklung zu erkennen. Die Aufarbeitung erfolgt nach dem Sauerstellen in üblicher Weise durch Extraktion. Das rohe Produkt der allgemeinen Formel (VI) kann durch Umkristallisieren gereinigt werden.

Der Reaktionsverlauf ist als äußerst überraschend zu bezeichnen, da eine Lactonbildung unter solch milden Bedingungen nicht bekannt ist.

Verwendet man beim Verfahren 10 zur Herstellung der Verbindungen der Formel (IX)

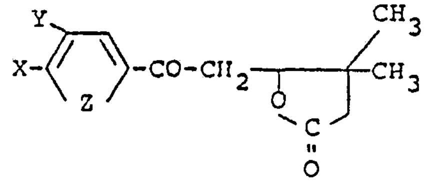

(IX)

Toluol und 3,3-Dimethyl-4-chlorcarbonylmethyl-γ-butyrolacton als Ausgangsstoffe, kann der Reaktionsablauf durch folgendes Formelschema wiedergegeben werden :

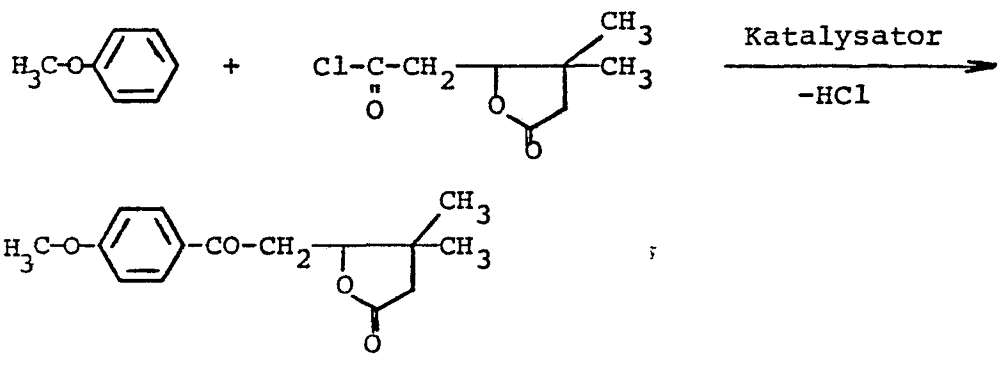


;

Die beim Verfahren 10 verwendbaren Ausgangsstoffe sind durch die Formeln (X) und (XI) definiert. Dei Verbindung (XI) ist neu ; ihre Herstellung wird weiter unten beschrieben.

Die Verbindungen der Formel (X) sind bekannt, beispielsweise seien genannt : Benzol, Fluorbenzol, Chlorbenzol, Brombenzol, Toluol, Ethylbenzol, Diphenylether, Xylol, o-Dichlorbenzol. Als Katalysatoren kommen grundsätzlich alle üblichen Friedel-Crafts-Katalysatoren in Frage, wie Aluminiumchlorid, Zinntetrachlorid, Titantetrachlorid, Fluorwasserstoff, Bortrifluorid, Eisen(III)-chlorid, Zinkchlorid, Polyphosphorsäuren, Perfluoralkansulfonsäuren (gegebenenfalls in polymerer Form), sowie gegebenenfalls Mischungen derselben.

Das Verfahren wird bevorzugt in Gegenwart eines Verdünnungsmittels durchgeführt. Es kommen in Frage : Methylenchlorid, Chloroform, Dichloräthan, Tetrachloräthan, Nitrobenzol, Nitromethan. Bevorzugt ist Methylenchlorid.

Die erfindungsgemäße Reaktion ist als äußerst überraschend zu bezeichnen, da erwartet werden mußte, daß auch der Lactonring in folgender Weise reagieren würde :

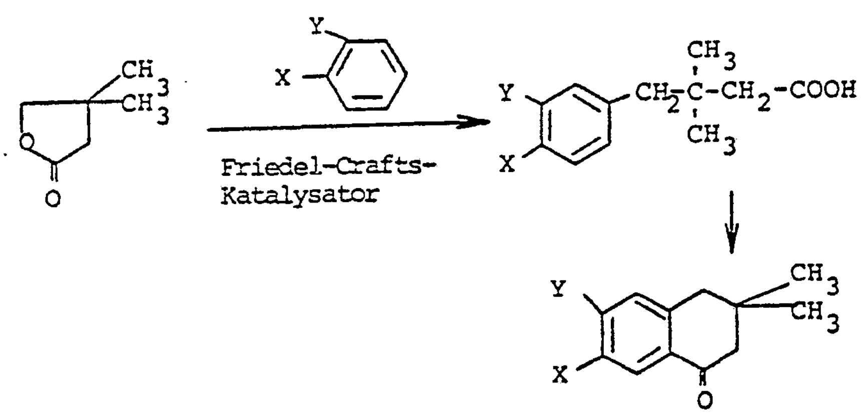


zusätzlich wäre der Ringschluß zum Tetralon zu erwarten gewesen. Ringöffnungen von 5-Ring-Lactonen mit Aromaten nach Friedel-Crafts in der oben formulierten Weise sind bekannt und erfolgen unter sehr milden Bedingungen (Houben-Weyl ; Band VI, 2 Seite 812 ff).

Daß eine solche Reaktion des Lactons mit den Aromaten der Formel (X) nicht erfolgt, ist vor allem auch deshalb überraschend, da der Friedel-Crafts-Katalysator mindestens äquimolar, besser noch im Überschuß, eingesetzt werden muß.

Bevorzugt wird Verfahren 10 wie folgt durchgeführt :

Man legt das Säurechlorid (XI) in einem Verdünnungsmittel vor und fügt bei Temperaturen zwischen − 10 und + 5 °C den Friedel-Crafts-Katalysator hinzu. Anschließend tropft man den Aromaten, gegebenenfalls ebenfalls in einem Verdünnungsmittel gelöst, zu. Verwendet man sehr wirksame Friedel-Crafts-Katalysatoren (z.B. Aluminiumchlorid, Zinntetrachlorid) geschieht dies bei − 10 bis + 5 °C, bei weniger

wirksamen Friedel-Crafts-Katalysatoren (z.B. Zinkchlorid, Eisenchlorid, Titantetrachlorid, Perfluoralkansulfonsäuren) tropft man den Aromaten bei Raumtemperatur zu. Anschließend rührt man bei Raumtemperatur nach ; bei weniger wirksamen Katalysatoren muß gegebenenfalls bei erhöhter Temperatur gearbeitet werden.

Verwendet man reaktionsträge Aromaten, wie beispielsweise Chlorbenzol, so empfiehlt es sich nicht, die Reaktion durch Temperaturerhöhung zu beschleunigen, sondern die Reaktionszeiten zu verlängern, um die unerwünschte Lactonringöffnung im oben erwähnten Sinn zu verhindern.

Die Aufarbeitung erfolgt in üblicher Weise ; die Lactone können durch Umkristallisieren gereinigt werden.

Die beim Verfahren 10 erhaltenen Verbindungen sind durch die Formel (IX) definiert. Darin steht X bevorzugt für Wasserstoff, Fluor, Chlor, Brom, Methyl, Äthyl, Phenyloxy, Methoxy, Äthoxy, Methylmercapto, Y steht bevorzugt für Wasserstoff, Chlor, Methyl, Methoxy oder X und Y für Methylendioxy oder Äthylendioxy und Z steht bevorzugt für O, S oder —CH = CH—.

Ganz besonders bevorzugt steht X für Wasserstoff, Fluor, Chlor, Brom, Methyl oder Methoxy, Y für Wasserstoff oder X und Y für Methylendioxy und Z für CH = CH.

Beispielsweise seien folgende Verbindungen der Formel (IX) genannt :

3,3-Dimethyl-4-phenacyl-γ-butyrolacton
3,3-Dimethyl-4-(4'-fluor-phenacyl)-γ-butyrolacton
3,3-Dimethyl-4-(4'-chlor-phenacyl)-γ-butyrolacton
3,3-Dimethyl-4-(4'-brom-phenacyl)-γ-butyrolacton
3,3-Dimethyl-4-(4'-methyl-phenacyl)-γ-butyrolacton
3,3-Dimethyl-4-(4'-methoxy-phenacyl)-γ-butyrolacton
3,3-Dimethyl-4-(4'-methylmercapto-phenacyl)-γ-butyrolacton
3,3-Dimethyl-4-(3',4'-methylendioxy-phenacyl)-γ-butyrolacton
3,3-Dimethyl-4-(3',4'-dimethoxy)-γ-butyrolacton.

Verfahren 11 kann durch folgende Reaktionsgleichung beschrieben werden :

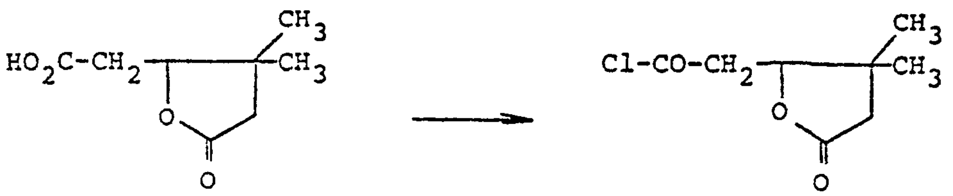

Die als Ausgangsstoff verwendete Säure der Formel (XII) ist bekannt (J. Org. Chem. ; Band 38, Seite 4148 und J. Chem. Soc. 79, 763), nicht jedoch das Säurechlorid der Formel (XI). Daß eine glatte Umwandlung der Säure in das Säurechlorid gelingt, erscheint überraschend, da unter diesen Bedingungen üblicherweise auch eine Ringöffnung des Lactons erfolgt.

Verfahren 11 wird unter den Bedingungen durchgeführt, die zur Herstellung eines Säurechlorids aus einer Säure üblich sind. Bevorzugte Chlorierungsmittel sind Thionylchlorid und Phosgen. Es ist jedoch darauf zu achten, daß möglichst kurze Reaktionszeiten angewendet werden, um eine Nebenreaktion im oben erwähnten Sinne zu vermeiden. Die Aufarbeitung erfolgt in üblicher Weise. Das Säurechlorid kann durch Destillation gereinigt oder roh in Verfahren 10 eingesetzt werden.

Beispiel 1 (Verfahren 3)

Zu einer Lösung von 35 g (0,1 Mol) 6-(4'-Chlor-phenyl)-4,6-dichlor-3,3-dimethyl-hex-5-en-säureäthylester in 100 ml Äthanol wird bei Raumtemperatur eine Lösung von natriumäthylat (hergestellt aus 2,5 g Natrium und 100 ml Äthanol) getropft. Man rührt 4 h nach, verdünnt mit Eiswasser und stellt neutral. Nach Extrahieren mit Methylenchlorid wird die organische Phase getrocknet und einrotiert. Durch Destillation im Hochvakuum erhält man 26,5 g eines schwach gelblichen Öls vom Siedepunkt 152-158 °C/0,08 mbar. Es enthält alle 4 möglichen Stereoisomeren des 2,2-Dimethyl-3-[2'-chlor-2'-(4'-chlorphenyl)]-vinyl-cyclopropan-1-carbonsäureäthylesters. Die Isomerenverteilung ist (Lt. Gaschromatogramm) : 66 % : 13,5 % : 8 % : 9 %. Das Vinylproton der Hauptisomeren zeigt (in $CDCl_3$) ein Dublett bei δ = 5,7 und 5,85 ppm. Massenspektrum : m/e = 312.

Beispiel 2 (Verfahren 3)

Eine Lösung von 40 g 6-(4'-Chlor-phenyl)-4,6,6-trichlor-3,3-dimethyl-hexansäureäthylester (roh ; aus Beispiel 8) in 100 ml Äthanol wird bei Raumtemperatur zu einer Lösung von 4,75 g Natrium in 250 ml Äthanol getropft. Man rührt 2 h bei Raumtemperatur nach und erhitzt anschließend noch 1 h auf 50 °C.

Dann wird mit Eiswasser versetzt und mit 10 %iger Salzsäure neutralisiert. Nach zweimaligem Extrahieren mit Methylenchlorid wird die organische Phase getrocknet, einrotiert und im Hochvakuum destilliert. Man erhält 25 g 2,2-Dimethyl-3-[2'-chlor-2'-(4'-chlorphenyl)] vinyl-cyclopropan-1-carbonsäureäthylester vom Siedepunkt 154-162 °C/0,1 mbar.

Beispiel 3 (Verfahren 3)

Eine Lösung von 32,7 g 6-(4'-Chlor-phenyl)-4,6,6-trichlor-3,3-dimethyl-hexansäurechlorid (roh, enthält noch $POCl_3$ ; aus Verfahren 8) in 500 ml Toluol wird unter Eiskühlung mit einer Lösung von 40 g Natrium in 700 ml Äthanol versetzt. Man rührt 8 h bei Raumtemperatur und erhitzt dann noch 1 h auf 50 °C, versetzt mit Eiswasser, neutralisiert mit 10 %iger Salzsäure, trennt die Toluolphase ab und extrahiert die Wasserphase mit Methylenchlorid. Die vereinigten organischen Phasen werden getrocknet und die Lösungsmittel sowie der gebildete Phosphorsäuretriäthylester abdestilliert. Der Rückstand wird durch Destillation im Hochvakuum gereinigt. Man erhält 19,6 g 2,2-Dimethyl-3-[2'-chlor-2'-(4'-chlor-phenyl)]-vinyl-cyclopropan-1-carbonsäureäthylester.

Beispiel 4 (Verfahren 3)

Analog Beispiel 3 erhält man aus 6-Phenyl-4,6,6-trichlor-3,3-dimethyl-hexansäurechlorid und Natriumäthylat 2,2-Dimethyl-3-[2'-chlor-2'-phenyl] vinyl-cyclopropan-1-carbonsäureäthylester.

Beispiel 5 (Verfahren 3)

Eine Lösung von 6,3 g 6-(4'-Chlor-phenyl)-4-chlor-3,3-dimethyl-hex-5-en-säureethylester in 30 ml Ethanol wird bei Raumtemperatur zu einer Lösung von Natriumethylat (hergestellt aus 0,7 g Natrium und 30 ml Ethanol) getropft. Man rührt 4 h nach, verdünnt mit Eiswasser und stellt neutral. Nach Extrahieren mit Methylenchlorid wird die organische Phase getrocknet und einsortiert und im Hochvakuum bei 60 °C andestilliert. Man erhält 4,6 g 2,2-Dimethyl-3-[2'-(4'-chlor-phenyl)-vinyl]-cyclopropan-1-carbonsäureethylester (Isomerengemisch).

Beispiel 6 (Verfahren $2a_1$)

Eine Lösung von 6-(4'-Chlor-phenyl)-4,6,6-trichlor-3,3-dimethyl-hexansäuremethylester wird in Toluol 1 h auf ca. 40 °C erwärmt. Nach Abdestillieren des Toluols im Vakuum bleibt 6-(4'-Chlor-phenyl)-4,6-dichlor-3,3-dimethyl-hex-5-en-säuremethylester zurück. Die Struktur wird durch das NMR-Spektrum bewiesen (olefinisches Proton bei 6,2 ppm in $CDCl_3$).

Beispiel 7 (Verfahren $2a_2$)

Eine Lösung von 6-(4'-Chlor-phenyl)-4,6,6-trichlor-3,3-dimethyl-hexancarbonsäurechlorid in Toluol (roh ; enthält noch $POCl_3$ ; aus Verfahren 7) wird mit der 20-fachen äquimolaren Menge trockenen Methanol bei 20 °C versetzt und anschließend noch 1 h auf 40 °C erwärmt. Nach 4 h Nachrühren (ohne Heizung) werden die Lösungsmittel und Phosphorsäureester in Vakuum entfernt. Der Rückstand ist identisch mit dem in Beispiel 6 erhaltenen Produkt.

Beispiel 8 (Verfahren $2a_3$)

Eine Lösung von 23,2 g 3,3-Dimethyl-4-(4'-chlor-phenacyl)-γ-butyrolacton in 400 ml Toluol wird mit 40 g Phosphorpentachlorid versetzt und solange bei Raumtemperatur gerührt, bis alles $PCl_5$ in Lösung gegangen ist. Anschließend werden bei 20 °C 120 ml Äthanol zugetropft, dann 1 h auf 45 °C erwärmt und noch solange nachgerührt, bis wieder Raumtemperatur erreicht ist. Anschließend wird auf viel Eiswasser gegeben und neutralgestellt. Die Toluolphase wird abgetrennt, getrocknet und einrotiert. Nach Abdestillieren der Lösungsmittel und des Phosphorsäuretriäthylester verbleiben 24 g eines dunklen Öls, das hauptsächlich aus 6-(4'-Chlor-phenyl)-4,6-dichlor-3,3-dimethyl-hex-5-en-säureäthylester besteht, das nach Verfahren 3 (Beispiel 1) weiterverarbeitet werden kann.

Beispiel 9 (Verfahren $2a_4$)

a) 26,6 g (0,1 Mol) des Lactons aus Beispiel 15 werden in 100 ml abs. Ethanol mit 1,13 g (30 mmol) Natriumborhydrid bei R.T. reduziert. Nach Aufarbeitung mit Methylenchlorid und verdünnter Salzsäure gewinnt man 28 g (100 %) 4,4-Dimethyl-dihydro-5-[2-(4-chlor-phenyl)-2-hydroxy-ethyl]-2-(3H)-furanon als Öl.

b) 28 g (0,1 Mol) Hydroxylacton (9a) werden mit 11,2 g (0,11 Mol) Acetanhydrid und einer Spur p-Toluol-sulfonsäure unter Erhitzen in 2 Std. zum Acetoxy-Derivat umgesetzt, das bei $Kp._{0,3}$ 198 °C bis

202 °C destilliert : 20,5 (72 %) Acetoxylacton.

c) Die Pyrolyse der Acetoxyverbindung (14,6 g) aus Beispiel 9 b in Toluollösung bei 400 °C über Glasringe im $N_2$-Strom führt zu 9,6 g (80 %) p-Chlorstyryllacton vom Fp. 64 °C-66 °C ($Kp._{0,1}$ 155-158 °C).

NMR ($CDCl_3$) : δ 1,05 (s, 3 H) ; 1,2 (s, 3 H) ; 2,45 (s, 2 H) ; 4,7 (d, J = 6 Hz, 1 H) ; 6,1 (dd, J = 6 u. 15 Hz, 1 H) ; 6,7 (d, J = 15 Hz, 1 H).

Aus dem Hydroxylacton 9 a kann bei einer Destillation an der Ölpumpe in Gegenwart von Oxalsäure direkt das p-Chlorstyryllacton erhalten werden.

d) 8 g 3,3-Dimethyl-4-[2' (4'-chlor-phenyl)-vinyl]-γ-butyrolacton werden in 50 ml Ethanol gelöst und trockener Chlorwasserstoff eingeleitet, bis die exotherme Reaktion abgeklungen ist. Nach Abdestillieren des Lösungsmittels verbleiben 8,8 g rohr 6-(4'-Chlor-phenyl)-4-chlor-3,3-dimethyl-hex-5-en-säureethylester, der direkt in Verfahren 3 (Beispiel 5) eingesetzt wird.

1 H-NMR ($CDCl_3$) : δ (ppm) = 1,0-1,3 (m, 9 H) ; 2,15-2,65 (g, 2 H) ; 3,9-4,3 (g, 2 H) ; 4,6-4,8 (d, 1 H) ; 5,98-6,78 (m, 2 H) ; 7,1-7,45 (m, 4 H).

Beispiel 10 (Verfahren 2b)

9,6 g 6-(4'-Chlor-phenyl)-6-oxo-4-chlor-3,3-dimethylhexansäureäthylester werden in 50 ml Toluol gelöst und bei Raumtemperatur 6,7 g Phosphorpentachlorid zugegeben. Man rührt 9 h bei Raumtemperatur nach, gibt auf 100 ml Eiswasser, stellt neutral und trennt die Toluolphase ab. Nach Trocknen und Abdestillieren des Toluols bei Raumtemperatur bleiben 8 g eines Öls zurück, das laut NMR-Spektrum aus 6-(4'-Chlor-phenyl)-4,6,6-trichlor-3,3-dimethyl-hexansäureäthylester besteht. Es kann nach Verfahren 3 weiter umgesetzt werden.

NMR ($CDCl_3$) : δ 1,0-1,3 (m, 9 H) ; 2,4 (m, 2 H) ; 2,9-3,5 (m, 2 H) ; 3,9-4,2 (m, 2 H) ; 4,6-4,8 (dd, 1 H) ; 7,3-8,0 (m, 4 H).

Beispiel 11 (Verfahren 7)

26,7 g 3,3-Dimethyl-4-(4'-chlor-phenyl)-γ-butyrolacton werden in 500 ml Toluol gelöst und mit 46 g Phosphorpentachlorid versetzt. Man rührt 18 h bei Raumtemperatur nach und destilliert dann bei Raumtemperatur im Vakuum Phosphoroxychlorid und Toluol ab. Es bleiben 38 g eines braunen Öls zurück, das durch IR und NMR als 6-(4'-Chlor-phenyl)-4,6,6-trichlor-3,3-dimethyl-hexancarbonsäurechlorid identifiziert wird.

Beispiel 12 (Verfahren 8)

Man löst 80,1 g 3,3-Dimethyl-4-(4'-chlor-phenacyl)-γ-butyrolacton in 500 ml Äthanol und leitet solange trockenen Chlorwasserstoff ein, bis 50 °C erreicht sind. Dann leitet man bei 50 °C zunächst unter Kühlung 3 h lang einen langsamen Chlorwasserstoffstrom durch die Lösung und leitet anschließend solange weiter ein, bis wieder Raumtemperatur erreicht ist. Durch Abdestillieren des Äthanols im Vakuum erhält man 100 g 6-(4'-Chlor-phenyl)-6-oxo-4-chlor-3,3-dimetnyl-hexansäureäthylester vom Brechungsindes $n_D^{20}$ = 1,528.

1H-NMR ($CDCl_3$) : δ = 1,05-1,4 ppm (9 H, m), 2,42 ppm (2 H) ; 3,25-3,55 ppm (2 H) ; 3,95-4,4 ppm (2 H) ; 4,6-4,85 (1 H) ; 7,3-8,05 ppm (m, 4 H).

Beispiel 13 (Verfahren 8)

Man löst 134 g 3,3-Dimethyl-4-(4'-chlor-phenacyl)-γ-butyrolacton in 215 g Thionylchlorid in einem 0,7 l Emaille-Autoklaven. Dann werden 100 g Äthanol zugepumpt und 4 h auf 50 °C gehalten. Nach Abkühlen und Entspannen destilliert man überschüssiges Thionylchlorid bzw. Schwefligsäureäthylester ab. Der Rückstand besteht im wesentlichen aus 6-(4'-Chlor-phenyl)-6-oxo-4-chlor-3,3-dimethyl-hexansäureäthylester.

Beispiel 14 (Verfahren 8)

Man löst 134 g 3,3-Dimethyl-4-(4'-chlor-phenacyl)-γ-butyrolacton in 215 g Thionylchlorid in einem 0,7 l Autoklaven mit Glaseinsatz. Dann werden 100 g Methanol zugepumpt und 4 h auf 50 °C erhitzt. Nach Aufarbeitung wie in Beispiel 10 erhält man 6-(4'-Chlor-phenyl)-6-oxo-4-chlor-3,3-dimethyl-hexansäuremethylester.

Beispiel 15 (Verfahren 9)

Zu der Mischung aus 87,5 g (0,5 mol) p-Chlorbenzoylchlorid und 130,3 g (0,5 mol) Zinntetrachlorid wurden 71 g (0,5 mol) 3,3-Dimethyl-4-pentensäuremethylester bei 20° unter Eiskühlung getropft. Nach dem Stehen über Nacht war die Reaktionsmischung erstarrt. Sie wurde in Methylenchlorid aufgenommen, filtriert und mit verd. Salzsäure ausgeschüttelt. Nach Trocknen und Abziehen des Lösungsmittels im

Vakuum erhält man 111,1 g Rohprodukt (ca. 83 %), das durchkristallisierte. Das Umkristallisieren aus 120 ml Ethanol lieferte 3,3-Dimethyl-4-(4'-chlor-phenacyl)-γ-butyrolacton vom Fp. 76-80 °C.

NMR ($CDCl_3$) : δ 1,15 (s, 3 H), 1,25 (s, 3 H), 2,45 (m, 2 H) 2,8-3,65 (8 l, 2 H), 4,9 (dd, 1 H), 7,3-8,05 (m, 4 H).

Beispiel 16 (Verfahren 9)

In eine Lösung von 17,5 g (0,1 mol) p-Chlorbenzoylchlorid und 14,2 g (0,1 mol) 3,3-Dimethyl-4-pentensäuremethylester in 100 ml Dichlormethan wurden bei 0° 26 g (0,1 mol) Zinntetrachlorid getropft und anschließend 8 Stunden bis zum Ende der Gasentwicklung unter Rückfluß erhitzt. Nach der Aufarbeitung mit verdünnter Salzsäure erhielt man 20,9 g Lacton, das dem in Beispiel 15 erhaltenen Produkt entsprach.

Beispiel 17 (Verfahren 9)

In eine Lösung von 14,1 g (0,1 mol) Benzoylchlorid und 14,2 g (0,1 mol) 3,3-Dimethyl-4-pentensäuremethylester in 100 ml Methylenchlorid wurden bei Raumtemperatur 26 g (0,1 mol) Zinntetrachlorid getropft und anschließend 8 h bis zum Ende der Gasentwicklung unter Rückfluß erhitzt. Nach Ansäuern mit verdünnter Salzsäure erhält man 21,6 g 3,3-Dimethyl-4-phenacyl-γ-butyrolacton vom Schmp. 86-88 °C.

Beispiel 18 (Verfahren 9)

Analog Beispiel 17 erhält man aus 4-Nitro-benzoylchlorid und 3,3-Dimethyl-4-pentensäuremethylester 3,3-Dimethyl-5-(4'-nitro-phenyl)-γ-butyrolacton vom Schmp. 107-110.

Beispiel 19 (Verfahren 9)

Analog Beispiel 17 wird erhalten :
3,3-Dimethyl-4-(4'-chlor-3'-nitro-phenyl)-γ-butyrolacton Schmp. 134-136 °C.

Beispiel 20 (Verfahren 11)

Man vermischt 17,2 g 3,3-Dimethyl-4-carboxymethyl-γ-butyrolacton mit 60 ml Thionylchlorid und erhitzt 1 h auf 80 °C. Dann destilliert man das überschüssige Thionylchlorid bei Normaldruck ab, die letzten Reste im Wasserstrahlvakuum. Der Rückstand besteht aus 3,3-Dimethyl-4-chlorcarbonylmethyl-γ-butyrolacton und kann direkt für Verfahren 10 verwendet werden. Es kann jedoch auch noch durch Destillation weiter gereinigt werden : Siedepunkt : 130-140 °C/0,3 mbar.

Beispiel 21 (Verfahren 10)

Man legt 80 g Aluminiumchlorid in 300 ml Methylenchlorid vor und tropft 59 g 3,3-Dimethyl-4-chlorcarbonylmethyl-γ-butyrolacton in 150 ml Methylenchlorid gelöst bei 0-5 °C zu. Anschließend tropft man 39,2 g Chlorbenzol in 50 ml Methylenchlorid gelöst ebenfalls bei 0-5 °C zu. Dann läßt man auf Raumtemperatur kommen und rührt noch 7 h bei Raumtemperatur nach. Nach Eingießen des Ansatzes in Eiswasser wird die organische Phase abgetrennt und neutral gewaschen. Nach Trocknen und Abdestillieren des Lösungsmittels erhält man 76 g rohes 3,3-Dimethyl-4-(4'-chlor-phenacyl)-γ-butyrolacton, das aus Äthanol umkristallisiert wird. Schmp. 78-80 °C.

Beispiele 22 und 23 (Verfahren 10)

Analog Beispiel 21 werden erhalten :
3,3-Dimethyl-4-(4'-methyl-phenacyl)-γ-butyrolacton ; Schmp. 96 °C.
3,3-Dimethyl-4-(phenacyl)-γ-butyrolacton ; Schmp. 86-88 °C.

**Ansprüche**

1. Verbindungen der Formel

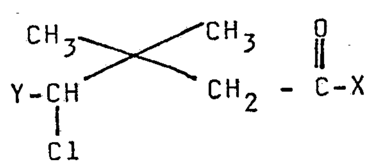

in welcher

Y für die Reste der Formeln

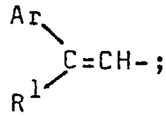 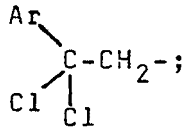 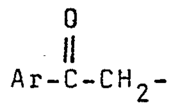

① ② ③

steht und

X für Cl oder —O—R steht für den Fall, daß Y für die Reste ① oder ② steht und für —O—R steht für den Fall, daß Y für den Rest ③ steht und

R für Alkyl oder für gegebenenfalls durch Halogen oder Cyano substituiertes 3-Phenoxy-benzyl steht und

$R^1$ für Wasserstoff, Fluor oder Chlor steht, und

Ar für gegebenenfalls durch Halogen, Cyano, Nitro, $C_{1-4}$-Alkyl, $C_{1-4}$-Alkoxy, $C_{1-4}$-Alkylthio, $C_{1-2}$-Halogenalkyl, $C_{1-4}$-Halogenalkoxy, $C_{1-4}$-Halogenalkylthio, gegebenenfalls halogensubstituiertes Alkylsulfonyl, Dialkylamino mit 1 oder 2 Kohlenstoffatomen in jedem Alkylsubstituenten oder gegebenenfalls halogensubstituiertes Methylendioxy oder Ethylendioxy substituiertes Phenyl steht.

2. Verfahren zur Herstellung von Verbindungen der Formel

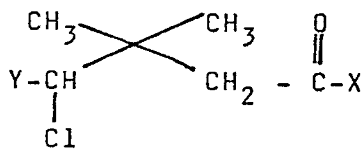

in welcher

Y für die Reste der Formeln

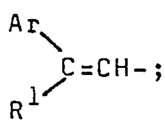 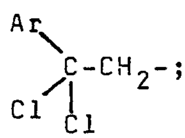 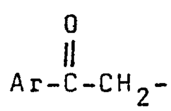

steht und

X für Cl oder —O—R steht für den Fall, daß Y für die Reste ① oder ② steht und für —O—R steht für den Fall, daß Y für den Rest ③ steht und

$R^1$ für Wasserstoff, Fluor oder Chlor steht und

Ar für gegebenenfalls durch Halogen, Cyano, Nitro, $C_{1-4}$-Alkyl, $C_{1-4}$-Alkoxy, $C_{1-4}$-Alkylthio, $C_{1-2}$-Halogenalkyl, $C_{1-4}$-Halogenalkoxy, $C_{1-4}$-Halogenalkylthio, gegebenenfalls halogensubstituiertes Alkylsulfonyl, Dialkylamino mit 1 oder 2 Kohlenstoffatomen in jedem Alkylsubstituenten oder gegebenenfalls halogensubstituiertes Methylendioxy oder Ethylendioxy substituiertes Phenyl steht, dadurch gekennzeichnet, daß man

a) für den Fall, daß Y für den Rest

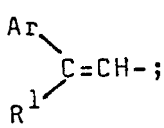

steht, worin

Ar und R die oben angegebene Bedeutung haben,

$a_1$) aus Verbindungen der obigen Formel, in denen Y für den Rest

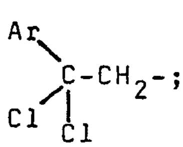

steht, worin

Ar die oben angegebene Bedeutung hat, thermisch Chlorwasserstoff abspaltet oder

$a_2$) Verbindungen der obigen Formel, in denen Y für den Rest

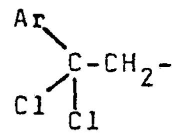

steht, worin

Ar die oben angegebene Bedeutung hat und

X für Cl steht, mit Alkoholen der Formel

R—O—H

worin

R die oben angegebene Bedeutung hat, umsetzt, oder

$a_3$) Verbindungen der Formel (VI)

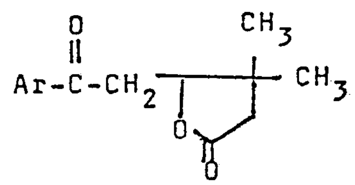

(VI)

worin

Ar die oben angegebene Bedeutung hat, mit mindestens 2 Äquivalent Phosphorpentachlorid umsetzt und anschließend mit einem Alkohol der Formel

R—OH

worin

R die oben angegebene Bedeutung hat, umsetzt oder

$a_4$) Verbindungen der Formel (VI) (oben) reduziert, Wasser abspaltet und mit einem Chlorierungsmittel in Gegenwart eines Alkohols der Formel

R—O—H

worin

R die oben angegebene Bedeutung hat, umsetzt, oder aber nacheinander zunächst den Lactonring der Verbindungen der Formel (VI) mit einem Chlorierungsmittel öffnet und anschließend mit einem Alkohol umsetzt oder

$a_5$) Verbindungen der Formel (V)

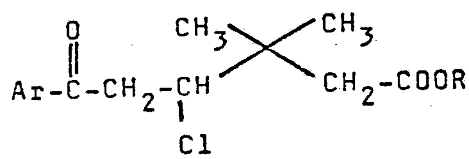

(V)

worin

Ar und R die oben angegebene Bedeutung haben, mit Schwefeltetrafluorid umsetzt, oder

$a_6$) Verbindungen, in denen Y für die Reste

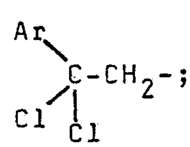

oder

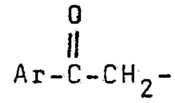

worin

Ar die oben angegebene Bedeutung hat, steht, mit wasserfreiem Fluorwasserstoff umsetzt und gegebenenfalls mit einem Alkohol der Formel

R—O—H

worin

R die oben angegebene Bedeutung hat, umsetzt, sowie

b) für den Fall, daß Y für den Rest

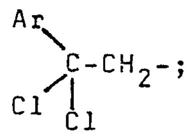

worin

Ar die oben angegebene Bedeutung hat,

steht und X für —O—R steht, (wobei R die oben angegebene Bedeutung hat), Verbindungen, in denen Y für den Rest

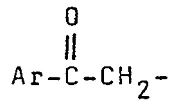

worin

Ar die oben angegebene Bedeutung hat, steht, mit Phosphorpentachlorid in einem Verdünnungsmittel unterhalb 30 °C umsetzt.

3. Verwendung von Verbindungen der Formel

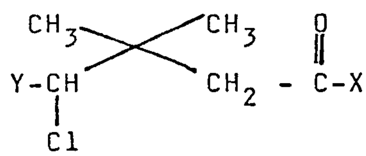

in welcher

X für Cl oder —O—R steht und

Y für die Reste der Formeln

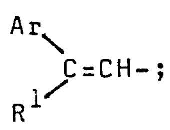
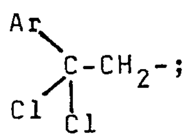
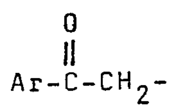

steht und

R für Alkyl oder für gegebenenfalls durch Halogen oder Cyano substituiertes 3-Phenoxy-benzyl steht und

Ar für gegebenenfalls durch Halogen, Cyano, Nitro, $C_{1-4}$-Alkyl, $C_{1-4}$-Alkoxy, $C_{1-4}$-Alkylthio, $C_{1-2}$-Halogenalkyl, $C_{1-4}$-Halogenalkoxy, $C_{1-4}$-Halogenalkylthio, gegebenenfalls halogensubstituiertes Alkylsulfonyl, Dialkylamino mit 1 oder 2 kohlenstoffatomen in jedem Alkylsubstituenten oder gegebenenfalls halogensubstituiertes Methylendioxy oder Ethylendioxy substituiertes Phenyl steht,

$R^1$ für Wasserstoff, Fluor oder Chlor steht, zur Herstellung von Cyclopropan-1-carbonsäureestern der allgemeinen Formel (I)

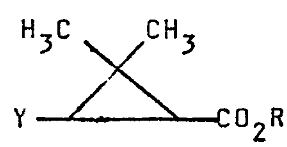
(I)

in der

Y für

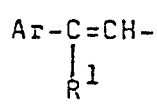

oder

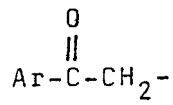

steht, und

Ar für gegebenenfalls durch Halogen, Cyano, Nitro, $C_{1-4}$-Alkyl, $C_{1-4}$-Alkoxy, $C_{1-4}$-Alkylthio, $C_{1-2}$-Halogenalkyl, $C_{1-4}$-Halogenalkoxy, $C_{1-4}$-Halogenalkylthio, gegebenenfalls halogensubstituiertes Alkylsulfonyl, Dialkylamino mit 1 oder 2 Kohlenstoffatomen in jedem Alkylsubstituenten oder gegebenenfalls halogensubstituiertes Methylendioxy oder Ethylendioxy substituiertes Phenyl steht,

R für Alkyl oder für gegebenenfalls durch Halogen oder Cyano substituiertes 3-Phenoxybenzyl steht und

$R^1$ für Wasserstoff, Fluor oder Chlor steht, dadurch gekennzeichnet, daß man Verbindungen der Formel

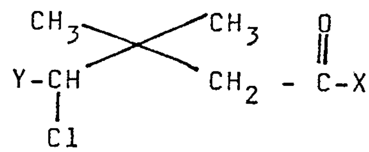

in welcher

Y und X die oben angegebene Bedeutung haben, mit einer äquivalenten Menge einer Base, gegebenenfalls in Gegenwart eines Verdünnungsmittels zwischen − 20 und + 60 °C umsetzt und gegebenenfalls mit einem Alkohol der Formel R—O—H umsetzt.

4. Verbindungen der Formel (II)

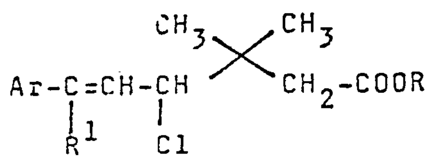

(II)

in welcher

Ar, R und $R^1$ die in Anspruch 1 angegebene Bedeutung haben.

5. Verbindungen der Formel (III)

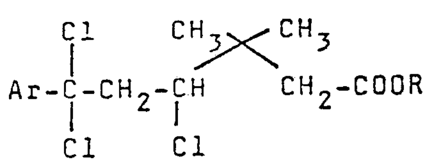

(III)

in welcher

Ar und R die in Anspruch 1 angegebene Bedeutung haben.

6. Verbindungen der Formel (IV)

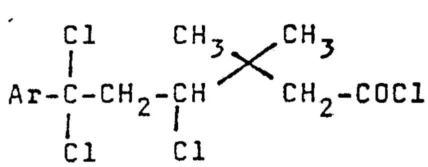

(IV)

in welcher

Ar die in Anspruch 1 angegebene Bedeutung hat.

**Claims**

1. Compounds of the formula

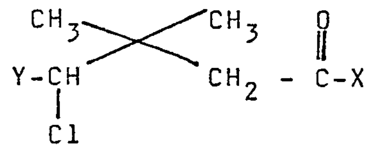

in which

Y represents radicals of the formulae

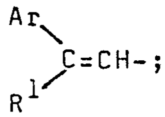 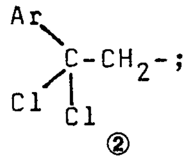 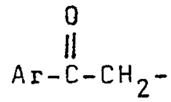

① ② ③

and

X represents Cl or —O—R in the case where Y represents the radicals ① or ② and represents —O—R in the case where Y represents the radical ③ and

R represents alkyl or 3-phenoxy-benzyl which is optionally substituted by halogen or cyano and

$R^1$ represents hydrogen, fluorine or chlorine, and

Ar represents phenyl which is optionally substituted by halogen, cyano, nitro, $C_{1-4}$-alkyl, $C_{1-4}$-alkoxy, $C_{1-4}$-alkylthio, $C_{1-2}$-halogenoalkyl, $C_{1-4}$-halogenoalkoxy, $C_{1-4}$-halogenoalkylthio, optionally halogen-substituted alkylsulphonyl, dialkylamino with 1 or 2 carbon atoms in each alkyl substituent or optionally halogen-substituted methylenedioxy or ethylenedioxy.

2. Process for the preparation of compounds of the formula

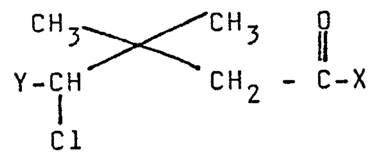

in which

Y represents the radicals of the formulae

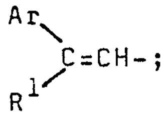 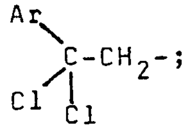 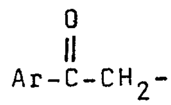

and

X represents Cl or —O—R in the case where Y represents the radicals 1 or 2 and represents —O—R in the case where Y represents the radical 3 and

$R^1$ represents hydrogen, fluorine or chlorine and Ar represents phenyl which is optionally substituted by halogen, cyano, nitro, $C_{1-4}$-alkyl, $C_{1-4}$-alkoxy, $C_{1-4}$-alkylthio, $C_{1-2}$-halogenoalkyl, $C_{1-4}$-halogenoalkoxy, $C_{1-4}$-halogenoalkylthio, optionally halogen-substituted alkylsulphonyl, dialkylamino with 1 or 2 carbon atoms in each alkyl substituent or optionally halogen-substituted methylenedioxy or ethylenedioxy, characterised in that

a) in the case where Y represents the radical

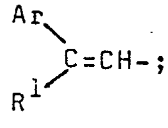

wherein

Ar and R have the meaning indicated above,

$a_1$) hydrogen chloride is split off, by the action of heat, from compounds of the above formula in Y represents the radical

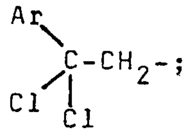

wherein

Ar has the meaning indicated above, or

$a_2$) compounds of the above formula, in which Y represents the radical

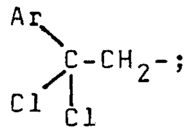

wherein

Ar has the meaning indicated above and

X represents Cl, are reacted with alcohols of the formula

R—O—H

wherein

R has the meaning indicated above, or

$a_3$) compounds of the formula (VI)

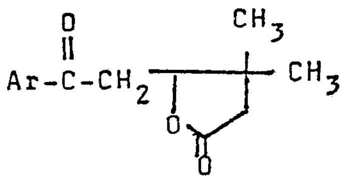

(VI)

wherein

Ar has the meaning indicated above are reacted with at least 2 equivalents of phosphorus pentachloride and the resulting reaction solution is then reacted with an alcohol of the formula

R—OH

wherein

R has the meaning indicated above, or

$a_4$) compounds of the formula (VI) (above) are reduced, water is split off from the compounds obtained and the products obtained are reacted with a chlorinating agent in the presence of an alcohol of the formula

R—O—H

wherein

R has the meaning indicated above, or, successively, the lactone ring of the compounds of the formula (VI) is first opened with a chlorinating agent and the product is then reacted with an alcohol or

$a_5$) compounds of the formula (V)

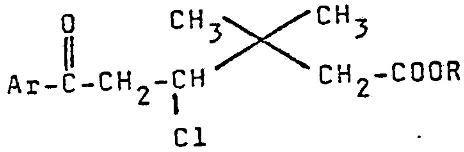

(V)

wherein

Ar and R have the meaning indicated above, are reacted with sulphur tetrafluoride, or

$a_6$) compounds, in which Y represents the radicals

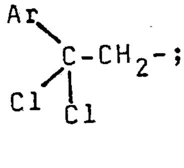

or

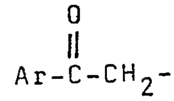

wherein

Ar has the meaning indicated above, are reacted with anhydrous hydrogen fluoride and, if appropriate, the products are reacted with an alcohol of the formula

R—O—H

wherein

R has the meaning indicated above, and

b) in the case where Y represents the radical

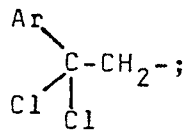

wherein

Ar has the meaning indicated above and X represents —O—R, (R having the meaning indicated above), compounds, in which Y represents the radical

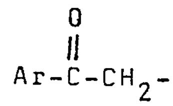

wherein

Ar has the meaning indicated above, are reacted with phosphorus pentachloride in a diluent below 30 °C.

3. Use of compounds of the formula

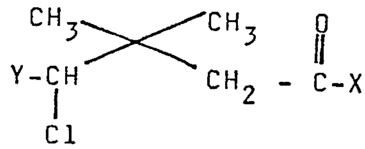

in which

X represents Cl or —O—R and

Y represents the radicals of the formulae

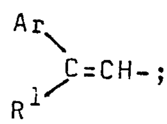

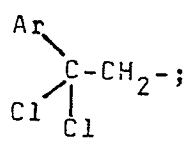

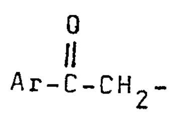

and

R represents alkyl or 3-phenoxy-benzyl which is optionally substituted by halogen or cyano and

Ar represents phenyl which is optionally substituted by halogen, cyano, nitro, $C_{1-4}$-alkyl, $C_{1-4}$-alkoxy, $C_{1-4}$-alkylthio, $C_{1-2}$-halogenoalkyl, $C_{1-4}$-halogenoalkoxy, $C_{1-4}$-halogenoalkylthio, optionally halogen-substituted alkylsulphonyl, dialkylamino with 1 or 2 carbon atoms in each alkyl substituent or optionally halogen-substituted methylenedioxy or ethylenedioxy, and

$R^1$ represents hydrogen, fluorine or chlorine, for the preparation of cyclopropane-1-carboxylic acid esters of the general formula (I)

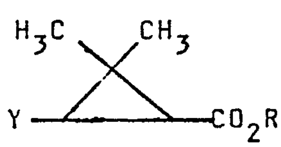

(I)

in which

Y represents

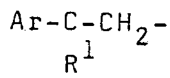

or

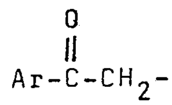

and

Ar represents phenyl which is optionally substituted by halogen, cyano, nitro, $C_{1-4}$-alkyl, $C_{1-4}$-alkoxy, $C_{1-4}$-alkylthio, $C_{1-2}$-halogenoalkyl, $C_{1-4}$-halogenoalkoxy, $C_{1-4}$-halogenoalkylthio, optionally halogen-substituted alkylsulphonyl, dialkylamino with 1 or 2 carbon atoms in each alkyl substituent or optionally halogen-substituted methylenedioxy or ethylenedioxy,

R represents alkyl or 3-phenoxy-benzyl which is optionally substituted by halogen or cyano and

$R^1$ represents hydrogen, fluorine or chlorine, characterised in that compounds of the formula

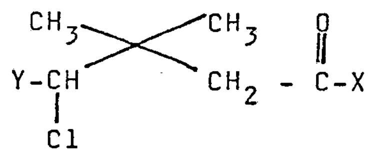

in which

Y and X have the meaning indicated above, are reacted with an equivalent amount of a base, if appropriate in the presence of a diluent, between − 20 and + 60 °C and if appropriate the product is reacted with an alcohol of the formula R—O—H.

4. Compounds of the formula (II)

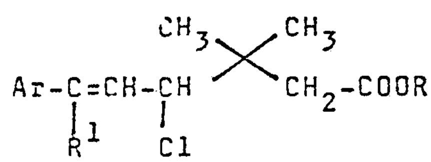

(II)

in which

Ar, R and $R^1$ have the meaning indicated in Claim 1.

5. Compounds of the formula (III)

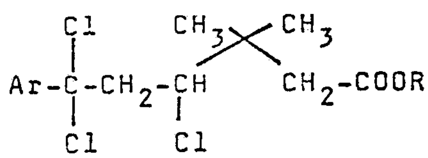

(III)

in which

Ar and R have the meaning indicated in Claim 1.

6. Compounds of the formula (IV)

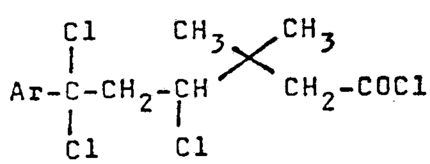

(IV)

in which

Ar has the meaning indicated in Claim 1.

**Revendications**

1. Composés de formule :

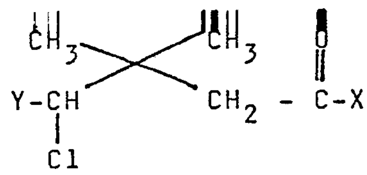

dans laquelle

Y représente les radicaux de formules :

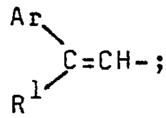 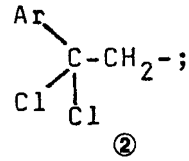 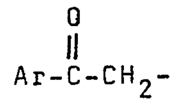

① ② ③

et

X représente Cl ou —O—R dans le cas où Y représente les radicaux ① ou ②, et —O—R dans le cas où Y représente le radical ③, et

R représente un alcoyle ou 3-phénoxy-benzyle éventuellement substitué par de l'halogène ou cyano et

$R^1$ représente de l'hydrogène, du fluor ou du chlore et

Ar représente un phényle éventuellement substitué par de l'halogène, cyano, nitro, alcoyle en $C_{1-4}$, alcoxy en $C_{1-4}$, alcoylthio en $C_{1-4}$, haloalcoyle en $C_{1-2}$, haloalcoxy en $C_{1-4}$, haloalcoylthio en $C_{1-4}$, alcoylsulfonyle éventuellement halo-substitué, dialcoylamino ayant 1 ou 2 atomes de carbone dans chaque substituant alcoyle, ou méthylènedioxy ou éthylènedioxy éventuellement halo-substitué.

2. Procédé de préparation de composés de formule :

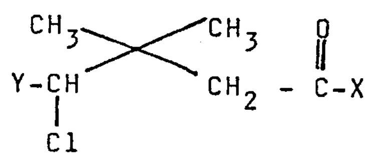

dans laquelle

Y représente les radicaux de formules :

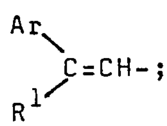 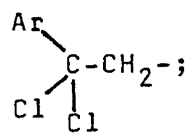 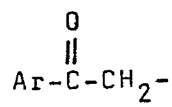

et

X représente Cl ou —O—R dans le cas où Y représente les radicaux 1 ou 2, et —O—R dans le cas où Y représente le radical 3, et

$R^1$ représente de l'hydrogène, du fluor ou du chlore et

Ar représente un phényle éventuellement substitué par de l'halogène, cyano, nitro, alcoyle en $C_{1-4}$, alcoxy en $C_{1-4}$, alcoylthio en $C_{1-4}$, haloalcoyle en $C_{1-2}$, haloalcoxy en $C_{1-4}$, haloalcoylthio en $C_{1-4}$, alcoylsulfonyle éventuellement halo-substitué, dialcoylamino ayant 1 ou 2 atomes de carbone dans chaque substituant alcoyle, ou méthylènedioxy ou éthylènedioxy éventuellement halo-substitué, caractérisé en ce que

a) dans le cas où Y représente le radical

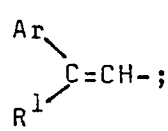

dans laquelle

Ar et R ont la signification indiquée plus haut,

$a_1$) on clive thermiquement de l'acide chlorhydrique à partir de composés de formule ci-dessus dans laquelle Y représente le radical

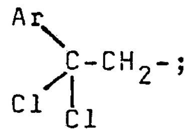

dans laquelle

Ar a la signification indiquée plus haut, ou

$a_2$) on fait réagir des composés de formule ci-dessus dans laquelle Y représente le radical

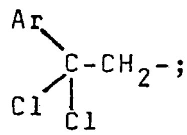

dans laquelle

Ar a la signification indiquée plus haut et X représente Cl, avec des alcools de formule

R—O—H

dans laquelle

R a la signification indiquée plus haut, ou

$a_3$) on fait réagir des composés de formule (VI)

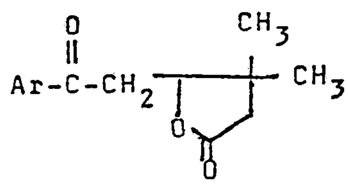

(VI)

dans laquelle

Ar a la signification indiquée plus haut, avec au moins 2 équivalents de pentachlorure de phosphore et l'on fait ensuite réagir avec un alcool de formule

R—OH

dans laquelle

R a la signification indiquée plus haut, ou

$a_4$) on réduit des composés de formule (VI) (ci-dessus), on clive de l'eau et l'on fait réagir avec un agent de chloruration en présence d'un alcool de formule

R—O—H

dans laquelle

R a la signification indiquée plus haut, ou bien, successivement, on ouvre d'abord le cycle lactone des composés de formule (VI) avec un agent de chloruration puis on fait réagir avec un alcool, ou

$a_5$) on fait réagir des composés de formule (V) :

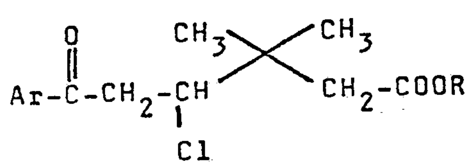

(V)

dans laquelle

Ar et R ont la signification indiquée plus haut, avec du tétrafluorure de soufre, ou

$a_6$) on fait réagir des composés dans lesquels Y représente les radicaux

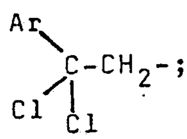

ou

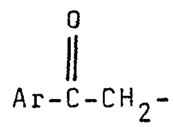

dans lesquels

Ar a la signification indiquée plus haut, avec de l'acide fluorhydrique anhydre et l'on fait réagir éventuellement avec un alcool de formule

R—O—H

dans laquelle

R a la signification indiquée plus haut, de même que

b) dans le cas où Y représente le radical

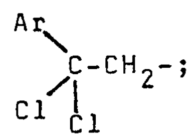

dans laquelle

Ar a la signification indiquée plus haut et X représente —O—R (R ayant la signification indiquée plus haut), on fait réagir des composés dans lesquels Y représente le radical

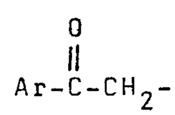

dans laquelle

Ar a la signification indiquée plus haut, avec du pentachlorure de phosphore dans un diluant au-dessous de 30 °C.

3. Utilisation de composés de formule

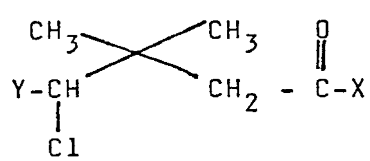

dans laquelle

X représente Cl ou —O—R et

Y représente les radicaux de formules

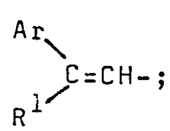 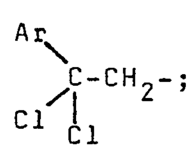 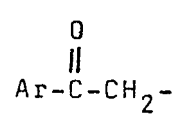

et

R représente un alcoyle ou un 3-phénoxy-benzyle éventuellement substitué par de l'halogène ou cyano et

Ar représente un phényle éventuellement substitué par de l'halogène, cyano, nitro, alcoyle en $C_{1-4}$, alcoxy en $C_{1-4}$, alcoylthio en $C_{1-4}$, haloalcoyle en $C_{1-2}$, haloalcoxy en $C_{1-4}$, haloalcoylthio en $C_{1-4}$, alcoylsulfonyle éventuellement halo-substitué, dialcoylamino ayant 1 ou 2 atomes de carbone dans chaque substituant alcoyle, ou méthylènedioxy ou éthylènedioxy éventuellement halo-substitué,

$R^1$ représente de l'hydrogène, du fluor ou du chlore, en vue de la préparation d'esters d'acides cycylopropane-1-carboxyliques de formule générale (I)

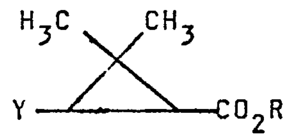

(I)

dans laquelle

Y représente 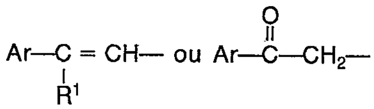

et

Ar représente un phényle éventuellement substitué par de l'halogène, cyano, nitro, alcoyle en $C_{1-4}$, alcoxy en $C_{1-4}$, alcoylthio en $C_{1-4}$, haloalcoyle en $C_{1-2}$, haloalcoxy en $C_{1-4}$, haloalcoylthio en $C_{1-4}$, alcoylsulfonyle éventuellement halo-substitué, dialcoylamino ayant 1 ou 2 atomes de carbone dans chaque substituant alcoyle, ou méthylènedioxy ou éthylènedioxy éventuellement halo-substitué,

R représente un alcoyle ou un 3-phénoxybenzyle éventuellement substitué par de l'halogène ou cyano et

$R^1$ représente de l'hydrogène, du fluor ou du chlore, caractérisée en ce qu'on fait réagir des composés de formule

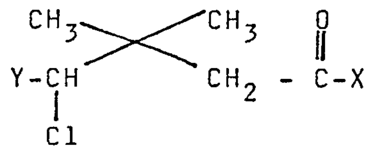

dans laquelle

Y et X ont la signification indiquée plus haut, avec une quantité équivalente d'une base, éventuellement en présence d'un diluant, entre − 20 et + 60 °C, et en ce qu'on fait éventuellement réagir avec un alcool de formule R—O—H.

4. Composés de formule (II)

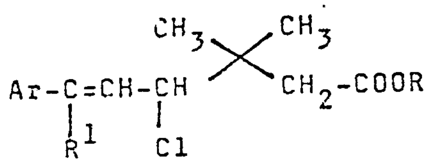 (II)

dans laquelle

Ar, R et $R^1$ ont la signification indiquée à la revendication 1.

5. Composés de formule (III)

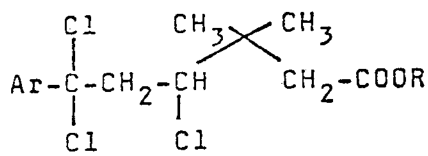 (III)

dans laquelle

Ar et R ont la signification indiquée à la revendication 1.

6. Composés de formule (IV)

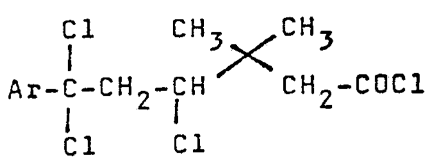 (IV)

dans laquelle

Ar a la signification indiquée à la revendication 1.